# EUROPEAN PATENT APPLICATION

(11) **EP 4 163 365 A1**
(43) Date of publication of application: **12.04.2023**
(21) Application number: 21822064.8
(22) Date of filing: 08.06.2021
(51) Int. Cl.: C12N 5/077, A61L 27/36, A61L 27/38

(54) **METHOD FOR PRODUCING CARDIOMYOCYTE POPULATION, METHOD FOR GROWING CARDIOMYOCYTES, CARDIOMYOCYTE POPULATION, GRAFT MATERIAL, MULTINUCLEAR CARDIOMYOCYTE, AND KIT FOR GROWING CARDIOMYOCYTES**

(30) Priority: 09.06.2020 JP 2020100045; 09.06.2020 JP 2020100063
(71) Applicant: Myoridge Co. Ltd., Kyoto-shi, Kyoto 606-8305 (JP)
(72) Inventor: NAKAMURA, Toyomi, Kyoto-shi, Kyoto 606-8305 (JP); FUJIKAWA, Saori, Kyoto-shi, Kyoto 606-8305 (JP); MINATOHARA, Maiko, Kyoto-shi, Kyoto 606-8305 (JP); MINAMI, Itsunari, Kyoto-shi, Kyoto 606-8305 (JP)
(74) Representative: AWA Sweden AB
(86) International application number: PCT/JP2021/021797
(87) International publication number: WO 2021/251393

(57) **Abstract**

A method of producing a cardiomyocyte population, the method comprising culturing a cell population containing cardiomyocytes seeded at a cell density of 0.1 × 10⁵ to 5.0 × 10⁵ cells/cm² in a culture solution to proliferate the cardiomyocytes.

SEQUENCE LISTING

<110>  Myoridge Co. Ltd.
<120>  Method of producing cardiomyocyte population,
method of proliferating cardiomyocytes, cardiomyocyte
population, transplantation material, multinucleated
cardiomyocyte, and kit for proliferating cardiomyocytes
<160>  4
<170>  PatentIn version 3.5

<210>  1
<211>  17
<212>  DNA
<213>  Artificial Sequence

<220>
<223>  Forward primer for Lin28
<400>  1
cacggtgcgg gcatctg                                                17

<210>  2
<211>  21
<212>  DNA
<213>  Artificial Sequence

<220>
<223>  Reverse primer for Lin28
<400>  2
ccttccatgt gcagcttact c                                           21

<210>  3
<211>  22
<212>  DNA
<213>  Artificial Sequence

<220>
<223>  Forward primer for cTnT

<400>  3
gaaggacctg aatgagttgc ag                                              22

<210>  4
<211>  23
<212>  DNA
<213>  Artificial Sequence

<220>
<223>  Reverse primer for cTnT

<400>  4
acgtsctctc gatcctgtct ttg                                             23

## Description

### FIELD

The present invention relates generally to a method of producing a cardiomyocyte population, a method of proliferating cardiomyocytes, a cardiomyocyte population, a transplantation material, a multinucleated cardiomyocyte, and a kit for proliferating cardiomyocytes.

### BACKGROUND

It has been common technical knowledge in this technical field that cardiomyocytes hardly proliferate even when cultured. Regarding the proliferation of cardiomyocytes, it has been reported that mouse cardiomyocytes and rat cardiomyocytes proliferate by a GSK-3 inhibitor (Non-Patent Literature 1 and Non-Patent Literature 2). It has been reported that human cardiomyocytes were proliferated up to about 1.5-fold by adding a combination of plural kinds of chemical substances to a culture solution (Non-Patent Literature 1) .

### CITATION LIST

### NON PATENT LITERATURES

Non-Patent Literature 1: Uosaki H et al., "Identification of Chemicals Inducing Cardiomyocyte Proliferation in Developmental Stage-Specific Manner With Pluripotent Stem Cells", Circ Cardiovasc Genet., 2013, 6(6), 624-633.
Non-Patent Literature 2: Tseng AS et al., "The GSK-3 Inhibitor BIO Promotes Proliferation in Mammalian Cardiomyocytes", Chemistry & Biology, 2006, 13, 957-963.

### SUMMARY

### TECHNICAL PROBLEM

It is an object of the present invention to provide a technique in which cardiomyocytes can be proliferated by a simple method.

### SOLUTION TO PROBLEM

According to first aspect, there is provided a method of producing a cardiomyocyte population, the method comprising culturing a cell population containing cardiomyocytes seeded at a cell density of 0.1 × 10⁵ to 5.0 × 10⁵ cells/cm² in a culture solution to proliferate the cardiomyocytes.

According to second aspect, there is provided a method of proliferating cardiomyocytes, the method comprising culturing a cell population containing cardiomyocytes seeded at a cell density of 0.1 × 10⁵ to 5.0 × 10⁵ cells/cm² in a culture solution to proliferate the cardiomyocytes.

According to third aspect, there is provided a cardiomyocyte population selected from the followings:
a cardiomyocyte population containing human multinucleated cardiomyocytes including three or more nuclei and derived from pluripotent stem cells,
a cardiomyocyte population containing human multinucleated cardiomyocytes with five or more nuclei, and
a cardiomyocyte population produced by the method according to first aspect.

According to fourth aspect, there is provided a transplantation material selected from the followings:
a transplantation material comprising a cardiomyocyte population containing human multinucleated cardiomyocytes including three or more nuclei and derived from pluripotent stem cells,
a transplantation material comprising a cardiomyocyte population containing human multinucleated cardiomyocytes with five or more nuclei, and
a transplantation material comprising a cardiomyocyte population produced by the method according to first aspect.

According to fifth aspect, there is provided a human multinucleated cardiomyocyte with five or more nuclei.

According to sixth aspect, there is provided a kit for proliferating cardiomyocytes, the kit comprising a cell population containing cardiomyocytes, a culture solution for cardiomyocytes, a WNT signal activator, and an EGF receptor inhibitor.

### ADVANTAGEOUS EFFECTS OF INVENTION

According to the present invention, cardiomyocytes can be proliferated by a simple method. The present invention is useful in any situation in which cardiomyocytes are provided, and particularly useful in a situation in which raw material cells for a transplantation material such as a myocardial sheet are provided.

### BRIEF DESCRIPTION OF THE DRAWINGS

FIG. 1 is a graph showing a cardiomyocyte proliferation rate.
FIG. 2 is a graph showing a ratio of multinucleated cardiomyocytes.
FIG. 3 is a graph showing an average maximum length of multinucleated cardiomyocytes.
FIG. 4 is a microscopic image showing an example of a cardiomyocyte population.
FIG. 5 is a microscopic image showing an example of a 3-nucleated cardiomyocyte.
FIG. 6 is a microscopic image showing an example of a 4-nucleated cardiomyocyte.
FIG. 7 is a microscopic image showing an example of a 7-nucleated cardiomyocyte.
FIG. 8 is a microscopic image showing sarcomeric structures in multinucleated cardiomyocytes.
FIG. 9 is a graph showing a ratio of multinucleated cardiomyocytes.
FIG. 10 is a graph showing an average maximum length of multinucleated cardiomyocytes.
FIG. 11 is a microscopic image showing a cardiomyocyte population obtained using commercially available cardiomyocytes.
FIG. 12 is a graph showing a cardiomyocyte purity.
FIG. 13 is a graph showing a ratio of multinucleated cardiomyocytes.
FIG. 14 is a graph showing an average maximum length of multinucleated cardiomyocytes.
FIG. 15A is a waveform diagram when terfenadine is not added.
FIG. 15B is a waveform diagram when terfenadine is added at a final concentration of 100 nM.
FIG. 15C is a waveform diagram when terfenadine is added at a final concentration of 300 nM.
FIG. 15D is a waveform diagram when terfenadine is added at a final concentration of 500 nM.
FIG. 15E is a waveform diagram when terfenadine is added at a final concentration of 900 nM.
FIG. 16A is a microscopic image showing the hearts of myocardial infarcted rats after transplantation.
FIG. 16B is a microscopic image showing the hearts of the myocardial infarcted rats after transplantation.
FIG. 17 is graphs showing expression levels in a cardiomyocyte marker gene (cTnT) and an undifferentiated iPS cell marker gene (Lin28).
FIG. 18 is a graph showing a detection limit of iPS cells.

### DETAILED DESCRIPTION

The present invention will be described below. The description provided below is intended to illustrate the present invention, and not intended to limit the present invention.

### 1. Method of Producing Cardiomyocyte Population

As described in the "Background" section, cardiomyocytes have been thought to be cells that hardly proliferate even when cultured. On the other hand, cells other than cardiomyocytes, such as fibroblasts and iPS cells, can proliferate through culturing. When proliferating cells that can proliferate through an adhesion culture, the cells are seeded in a culture solution at a relatively low cell density so as to ensure a space in which the cells can proliferate. After that, the cell proliferation stops when the cells proliferate and reach a confluent state. For example, the literature by Uosaki H et al. cited in the Background section describes that a low cell density (5.0 × 10⁴/well [6-well plate], i.e., about 0.5 × 10⁴ cells/cm²) was employed to proliferate mouse ES cell-derived cardiomyocytes.

In contrast to such common technical knowledge, the present inventors have found that only cardiomyocytes can proliferate when being seeded at a relatively high cell density at which a sufficient space for proliferation is not secured, and have developed the present invention.

That is, the method of producing a cardiomyocyte population includes culturing a cell population containing cardiomyocytes seeded at a density of 0.1 × 10⁵ to 5.0 × 10⁵ cells/cm² in a culture solution to proliferate the cardiomyocytes.

More specifically, the method of producing a cardiomyocyte population can include
seeding a cell population containing cardiomyocytes in a culture vessel in a state of being suspended in a culture solution so as to have a cell density of 0.1 × 10⁵ to 5.0 × 10⁵ cells/cm², and
culturing the cell population in the culture solution to proliferate the cardiomyocytes.

### (Cell Population Containing Cardiomyocytes)

First, seeded cells, i.e., a "cell population containing cardiomyocytes" will be described.

The "cell population containing cardiomyocytes" is a cell population prepared as cardiomyocytes, but may contain cells other than cardiomyocytes. A ratio of cardiomyocytes contained in the cell population (i.e., a cardiomyocyte purity) is preferably 80 to 100%, and more preferably 90 to 100%. In the present specification, the cardiomyocyte purity represents a ratio (percentage) of the number of cardiomyocytes if the number of total cells is taken as 100. The cardiomyocyte purity can be determined by flow cytometry analysis using antibodies such as cardiac troponin T (cTnT), α-actinin, α-cardiac actin, and GATA-4, which are cardiac markers. Since the cardiomyocyte purity of the cell population can be increased by culturing the cell population according to the above method, it is not a problem if the cell population has a relatively low cardiomyocyte purity at the start of culturing.

The "cell population containing cardiomyocytes" is, for example, a cell population obtained by inducing differentiation of pluripotent stem cells into cardiomyocytes. Alternatively, the "cell population containing cardiomyocytes" may be primary cultured cardiomyocytes isolated from the heart of an organism. Alternatively, the "cell population containing cardiomyocytes" may be commercially available pluripotent stem cell-derived cardiomyocytes (e.g., iCell of CDI, MiraCell of Takara Bio Inc., Cor.4U of Axiogenesis, etc.).

The "pluripotent stem cell" refers to a cell having pluripotency, which is an ability to differentiate into any type of cell constituting an adult body and a self-renewal potential, which is an ability to maintain pluripotency even after cell division. The "pluripotent stem cell" includes an embryonic stem cell (an ES cell), an embryonic germ cell (an EG cell), and an induced pluripotent stem cell (an iPS cell).

The "cell population containing cardiomyocytes" is preferably a cell population obtained by inducing differentiation of iPS cells into cardiomyocytes (hereinafter also referred to as iPS cell-derived cardiomyocytes). The iPS cell-derived cardiomyocytes can be prepared by a known method of inducing cardiomyocyte differentiation such as a method of inducing protein-free cardiomyocyte differentiation (PFCD) (see WO 2015/182765). The method of inducing protein-free cardiomyocyte differentiation (PFCD) can produce a cell population containing cardiomyocytes with high purity, for example, a cell population containing cardiomyocytes with a purity of 80 to 100%.

The "cell population comprising cardiomyocytes" may be a cell population containing cardiomyocytes of any organism, but is preferably a cell population containing cardiomyocytes of a mammal, and more preferably a cell population containing cardiomyocytes of a human.

As described above, the "cell population containing cardiomyocytes" is preferably a cell population obtained by inducing differentiation of human iPS cells into cardiomyocytes (hereinafter also referred to as human iPS cell-derived cardiomyocytes). And more preferably, the "cell population containing cardiomyocytes" is a cell population obtained by inducing differentiation of human iPS cells into mature cardiomyocytes (hereinafter also referred to as human iPS cell-derived mature cardiomyocytes). Since a degree of maturity of cardiomyocytes can be increased by culturing a cell population according to the above-described method, the cell population may contain immature cardiomyocytes at the start of culturing.

The term "human iPS cell-derived mature cardiomyocytes" is used in contrast with the term "human iPS cell-derived immature cardiomyocytes" in this technical field, and the human iPS cell-derived mature cardiomyocytes have a higher ion channel function than the human iPS cell-derived immature cardiomyocytes.

The "human iPS cell-derived mature cardiomyocytes" are, for example, cells present after 14 or more days from the start of induction of differentiation of iPS cells. The day when the induction of differentiation of the iPS cells is started is the day when iPS cells maintained in an undifferentiated state are subjected to treatment for transferring to a differentiated state, and is set to be the 0th day. Since the "human iPS cell-derived mature cardiomyocytes" can be cultured for a long period of time while maintaining the differentiated state, an upper limit of the number of days elapsed from the start of differentiation induction in the "human iPS cell-derived mature cardiomyocytes" is not particularly limited. That is, the "human iPS cell-derived mature cardiomyocytes" may be cultured or stored for a long period of time while maintaining their differentiated state, and then used as seeding cells. For example, the "human iPS cell-derived mature cardiomyocytes" may be cells present after 365 days or more from the start of induction of differentiation of iPS cells. The "human iPS cell-derived mature cardiomyocytes" preferably refer to cells present after 14 days or more (e.g., 30 to 37 days) from the start of induction of differentiation of iPS cells by the method of inducing protein-free cardiomyocyte differentiation (PFCD).

### (Seeding)

In this method, a "cell population containing cardiomyocytes" is seeded at a cell density of 0.1 × 10⁵ to 5.0 × 10⁵ cells/cm². The "cell population containing cardiomyocytes" is seeded at a cell density of preferably 2.0 × 10⁵ to 5.0 × 10⁵ cells/cm², and more preferably 2.0 × 10⁵ to 4.0 × 10⁵ cells/cm².

The above-described cell density is higher than a seeding density generally used to proliferate and culture cells other than cardiomyocytes. The cell density of 5.0 × 10⁵ cells/cm² is close to an upper limit of the cell density that allows for seeding. Thus, if the "cell population containing cardiomyocytes" is seeded at a relatively high cell density, the cardiomyocytes can proliferate efficiently. In this case, the cardiomyocytes can proliferate so as to rise in a depth direction of a culture solution in a state of adhering to a bottom surface even though a sufficient space for proliferation is not secured.

Typically, seeding can be performed by seeding a cell population containing cardiomyocytes in a culture vessel in a state of being suspended in a culture solution so as to have a cell density of 0.1 × 10⁵ to 5.0 × 10⁵ cells/cm². The "cell population containing cardiomyocytes" is usually seeded in a single-cell state. Cells in a single-cell state can be prepared by, for example, treating a cell mass with a proteolytic enzyme (e.g., trypsin) .

As the culture solution, a publicly known culture solution for cardiomyocytes can be used. The culture solution preferably contains inorganic salts including calcium salts, magnesium salts, potassium salts, and sodium salts, and a buffer solution, as basic components. A basal medium such as DMEM, RPMI, IMDM, Ham-12, or the like may be used as the culture solution. The basal medium is available from, for example, Sigma-Aldrich Japan.

For example, a mixture having the following composition of basic components may be used in the culture solution for cardiomyocytes:
0.01 to 0.5 g/L (e.g., 0.182 g/L) of CaCl₂,
0 to 1.0 g/L (e.g., 0.09767 g/L) of MgSO₄,
0.1 to 1.0 g/L (e.g., 0.4 g/L) of KCl,
0 to 10.0 g/L (e.g., 3.362 g/L) of NaHCO₃,
1.0 to 20.0 g/L (e.g., 5.4525 g/L) of NaCl,
0 to 1.0 g/L (e.g., 0.109 g/L) of Na₂HPO₄, and
0 to 20.0 g/L (e.g., 5.958 g/L) of HEPES.

The culture solution may contain, in addition to the basic components, conventional components conventionally added to cell culture solutions. Examples of the conventional components include serum and a ROCK inhibitor.

That is, the culture solution may contain serum. The serum is known to enhance the proliferation rate of cells. The serum is, for example, fetal bovine serum (FBS). When the culture solution contains serum, the serum can be contained in an amount of 0.1 to 50% by mass in the culture solution before addition of the conventional component. The serum has an effect of increasing the proliferation rate of cardiomyocytes, but if the serum concentration is high, the proliferation rate of cells other than cardiomyocytes is also increased, so the concentration in the above range is preferable.

In addition, the culture solution may contain a ROCK inhibitor. The ROCK inhibitor is known to suppress cell death that can occur when cells are seeded in a single-cell state. The ROCK inhibitor refers to a substance that inhibits Rho kinase, and examples thereof include Y-27632 ((R)-(+)-trans-4-(1-aminoethyl)-N-(4-pyridyl) cyclohexanecarboxamide dihydrochloride; CAS. No. 129830-38-2). When the culture solution contains a ROCK inhibitor, the ROCK inhibitor can be contained at a concentration of 0.1 to 100 µM. Therefore, the ROCK inhibitor is preferably contained in the culture solution containing cells to be seeded, but may not be contained in a new culture solution to be used for culture solution exchange.

The culture solution may further contain additional components specific to the present invention. That is, the culture solution may contain a component that promotes the proliferation of cardiomyocytes as an additional component specific to the present invention. An example of a component that promotes the proliferation of cardiomyocytes is a WNT signal activator, and an embodiment using the WNT signal activator will be described later as a preferred embodiment.

In addition, when cells other than cardiomyocytes (e.g., fibroblasts) are contained in the cell population before culturing, the culture solution may contain a component that suppresses proliferation of the cells other than cardiomyocytes as an additional component specific to the present invention. An example of the component that suppresses proliferation of cells other than cardiomyocytes is an EGF receptor inhibitor, and an embodiment in which the EGF receptor inhibitor is used will be described later as a preferred embodiment.

A pH of the culture solution is not particularly limited, but is preferably 6.0 to 9.0, and more preferably 7.0 to 8.0.

As the culture vessel, any vessel used for an adhesion culture of cells can be used. Generally, a flat-bottom vessel such as a culture flask, a culture dish, a culture plate, or the like can be used as the culture vessel. The culture vessel may be coated with one or more substances capable of promoting cardiomyocyte adhesion, proliferation, and/or maturation. Such substances include extracellular matrices, e.g., gelatin, laminin, fibronectin, collagen, vitronectin, matrigel, etc. As an example, the culture vessel can be prepared by commercially obtaining a culture vessel coated with gelatin and coating this culture vessel with laminin. For example, iMatrix-511 (Nippi, Incorporated) can be used as laminin.

### (Culturing)

A cell population containing cardiomyocytes can be seeded at a cell density of 0.1 × 10⁵ to 5.0 × 10⁵ cells/cm² and then cultured in a culture solution to proliferate the cardiomyocytes. The culturing is preferably performed through an adhesion culture.

The culturing can be performed under a condition suitable for culturing the cardiomyocytes. The culturing can be performed, for example, in an atmosphere of 5% CO2 at a temperature of 30 to 43 °C over a discretionary period (e.g., 1 to 1000 days). During the culturing, the culture solution may be exchanged as necessary (e.g., every 1 to 14 days).

The culturing is preferably performed under a condition in which a distance from a liquid surface of the culture solution to the culture vessel bottom surface in contact with the cardiomyocytes (hereinafter also referred to as a liquid-surface distance) is 3 to 30 mm. The liquid-surface distance is more preferably from 5 to 10 mm. For example, when a T25 flask (area: 25 cm²) is used as the culture vessel, the culturing is preferably performed in a state in which 12.5 to 25 mL of the culture solution is placed in the culture vessel. As described above, it is preferable for the proliferation of cardiomyocytes that the cardiomyocytes be cultured at a constant liquid-surface distance.

In order to maximize the effect of proliferating cardiomyocytes, it is desirable to select a culture condition suitable for the proliferation of cardiomyocytes, and it is desirable to appropriately select, for example, a culture temperature, a timing of exchanging the culture solution, a liquid-surface distance, etc. within the above-described ranges.

When a cell population containing cardiomyocytes is cultured according to the above method, the cardiomyocytes can be proliferated. Thereby, a cell population containing the proliferated cardiomyocytes (hereinafter, also referred to as a cardiomyocyte population) can be produced. The above method is a simple and low-cost method because cardiomyocytes can be proliferated only by seeding cells at an appropriate cell density.

When cells other than cardiomyocytes (e.g., fibroblasts) are contained in the cell population, the cells other than cardiomyocytes also proliferate. However, the cells other than cardiomyocytes easily proliferate when seeded at a low cell density, but do not easily proliferate when seeded at a relatively high cell density as in the above method. Therefore, a cardiomyocyte population produced according to the above method can have a cardiomyocyte purity comparable to or higher than that of a cell population prior to culturing.

### (Serial Subculture)

A part of the cardiomyocyte population obtained through the above culturing may be suspended in a new culture solution and cultured for the second and subsequent passages. That is, the culturing in the above method may be performed through a serial subculture. The serial subculture can be performed by repeating the above culturing. Specifically, the culturing of the second and subsequent passages can be performed by bringing the cardiomyocyte population obtained through the previous culturing into a single-cell state, suspending it in a new culture solution so as to have a cell density of 0.1 × 10⁵ to 5.0 × 10⁵ cells/cm², seeding the obtained cell suspension in a culture vessel, and culturing the cells.

In the culturing of the second and subsequent passages, the same cell density as that in the previous culturing may be employed, or a cell density different from that in the previous culturing may be employed as long as the cell density is within the range of 0.1 × 10⁵ to 5.0 × 10⁵ cells/cm². In the culturing of the second and subsequent passages, the cardiomyocyte population obtained through the previous culturing is seeded at a cell density of preferably 2.0 × 10⁵ to 5.0 × 10⁵ cells/cm², and more preferably 2.0 × 10⁵ to 4.0 × 10⁵ cells/cm².

When the serial subculture is performed, the cardiomyocytes can be further proliferated. For example, when the cardiomyocytes proliferate at a proliferation rate of 2-fold after the culturing of the first passage and proliferate at a proliferation rate of 1.5-fold after the culturing of the second passage, a total proliferation rate of 3-fold can be achieved. The "total proliferation rate" can be calculated by multiplying all the proliferation rates obtained in the culturing of the respective passages. Thus, by performing the serial subculture, the proliferated cardiomyocytes can be further proliferated, and a high total proliferation rate can be achieved.

However, as the number of passages is increased, the cardiomyocyte proliferation rate obtained through culturing of each passage tends to decrease. It is considered that this is because as the culturing is continued, the cardiomyocytes become mature or multinucleate and become difficult to proliferate. Therefore, the serial subculture can be performed preferably over 2 to 5 passages, and more preferably over 2 to 4 passages.

### (Preferred Embodiment)

In the following, a preferred embodiment of the above-described method will be described. In the following description, only portions characteristic of the preferred embodiment will be described, and description of portions overlapping with the description of the above method will be omitted.

### <Preferred Embodiment: Embodiment using WNT Signal Activator>

In the above-described method, the culture solution preferably contains a WNT signal activator. That is, in a preferred embodiment, a method of producing a cardiomyocyte population includes culturing a cell population containing cardiomyocytes seeded at a cell density of 0.1 × 10⁵ to 5.0 × 10⁵ cells/cm² in the culture solution containing the WNT signal activator to proliferate the cardiomyocytes.

More specifically, the method of producing a cardiomyocyte population can include
seeding a cell population containing cardiomyocytes in a culture vessel in a state of being suspended in a culture solution so as to have a cell density of 0.1 × 10⁵ to 5.0 × 10⁵ cells/cm², and
culturing the cell population in a culture solution containing a WNT signal activator to proliferate the cardiomyocytes.

The WNT signal activator may be added to the cell suspension prior to seeding or may be added to the cell suspension after seeding.

The WNT signal activator refers to a substance that activates a WNT signaling pathway, and specific examples thereof include GSK3β inhibitors such as CHIR99021, BIO, TWS119, Kenpaullone, TCS 2002, TC-G 24, SB 415286, NSC 693868, A 1070722, AR-A014418, lithium chloride, etc.

The WNT signal activator is preferably CHIR99021. CHIR99021 is 6-[2-[[4-(2,4-dichlorophenyl)-5-(5-methyl-1H-imidazol-2-yl) pyrimidin-2-yl]amino]ethylamino]pyridine-3-carbonitrile with CAS number of 252917-06-9.

The WNT signal activator can be contained in a culture solution at a concentration of preferably 1 to 5 µM, more preferably 2 to 5 µM, and still more preferably 2 to 4 µM.

When a culture solution containing the WNT signal activator is used, the proliferation rate of cardiomyocytes can be further increased. A mechanism by which the WNT signal activator promotes the proliferation of cardiomyocytes is considered as follows. It is generally known that a WNT signal is a cell signal involved in cell proliferation and canceration. In addition, cardiomyocytes require WNT signal activation in order to differentiate into progenitor cells through mesoderm induction during their development, whereas inhibition of WNT signals is required in a process of differentiation from mesoderm into myocardium. This suggests that cardiomyocytes are highly sensitive to WNT signals. Therefore, since cardiomyocytes are more likely to undergo cell proliferation by WNT signals than other cells such as fibroblasts under an appropriate condition, it is considered that selective activation of WNT signals allows efficient cell proliferation restricted to cardiomyocytes.

When a culture solution containing the WNT signal activator is used, the "cell population containing cardiomyocytes" is seeded at a cell density of preferably 0.3 × 10⁵ to 4.0 × 10⁵ cells/cm², and more preferably 1.0 × 10⁵ to 3.0 × 10⁵ cells/cm².

When a culture solution contains the WNT signal activator as well, the culturing may be performed through a serial subculture. In the culturing of the second and subsequent passages, the same cell density as that in the previous culturing may be employed, or a cell density different from that in the previous culturing may be employed as long as the cell density is within the range of 0.1 × 10⁵ to 5.0 × 10⁵ cells/cm². In the culturing of the second and subsequent passages, the cardiomyocyte population obtained in the previous culturing is seeded at a cell density of preferably 0.3 × 10⁵ to 4.0 × 10⁵ cells/cm², and more preferably 1.0 × 10⁵ to 3.0 × 10⁵ cells/cm².

Thus, by combining "seeding of cells at an appropriate cell density" and "addition of a WNT signal activator to a culture solution", the proliferation rate of cardiomyocytes can be significantly increased. In Example 2 to be described later, it is demonstrated that a total proliferation rate of 20-fold or more can be achieved by performing serial subcultures of the third to fourth passages according to this embodiment. The total proliferation rate achieved in this embodiment far exceeds previously reported cardiomyocyte proliferation rates.

In addition, in this embodiment, since the proliferation rate of cardiomyocytes can be significantly increased, the proliferation rate of cardiomyocytes can be increased as compared with the proliferation rate of cells other than cardiomyocytes (e.g., fibroblasts). Therefore, in this embodiment, a ratio of the proliferated cardiomyocytes in the cell population (i.e., cardiomyocyte purity) can be increased.

### <Preferred Embodiment: Embodiment Not Using WNT Signal Activator>

Alternatively, in the above method, the culture solution may not contain a WNT signal activator. When the culture solution does not contain a WNT signal activator, a culture solution generally used for cell culturing can be used, and for example, the culture solution can be composed of the above-described basic components (specifically, inorganic salts and a buffer solution) and, if necessary, the above-described conventional components (specifically, serum and a ROCK inhibitor).

When the culture solution does not contain a WNT signal activator, a "cell population containing cardiomyocytes" is seeded at a cell density of preferably 2.0 × 10⁵ to 5.0 × 10⁵ cells/cm², and more preferably 2.0 × 10⁵ to 4.0 × 10⁵ cells/cm². When the culture solution does not contain a WNT signal activator, the higher the cell density at the time of seeding within the above range, the more the cardiomyocyte proliferation rate can be increased.

This embodiment is a particularly simple and low-cost method because it does not require the use of a culture solution having a composition specific to the present invention and can be performed only by adjusting the seeding density of cells.

### <Preferred Embodiment: Embodiment Using EGF Receptor Inhibitor>

In the above-described method, the culture solution preferably contains an EGF receptor inhibitor. That is, in a preferred embodiment, a method of producing a cardiomyocyte population includes culturing a cell population containing cardiomyocytes seeded at a cell density of 0.1 × 10⁵ to 5.0 × 10⁵ cells/cm² in a culture solution containing an EGF receptor inhibitor to proliferate the cardiomyocytes.

More specifically, the method of producing a cardiomyocyte population can include
seeding a cell population containing cardiomyocytes in a culture vessel in a state of being suspended in a culture solution so as to have a cell density of 0.1 × 10⁵ to 5.0 × 10⁵ cells/cm², and
culturing the cell population in a culture solution containing an EGF receptor inhibitor to proliferate the cardiomyocytes.

The EGF receptor inhibitor may be added to the cell suspension before seeding or may be added to the cell suspension after seeding.

The EGF receptor inhibitor refers to a substance that inhibits signaling transduction from an EGF receptor, and examples thereof include AG1478, gefitinib, Afatinib, ARRY334543, AST 1306, AZD8931, BIBU 1361, BIBX 1382, BPDQ, BPIQ-I, BPIQ-II, Canertinib, CL-387,785, CUDC 101, Dacomitanib, Vandetanib, EGFR Inhibitor III, EGFR/ErbB-2 Inhibitor, Erlotinib, GW 583340, GW2974, HDS 029, Lapatinib, WHI-P 154, OSI-420, PD 153035, PD 168393, PD 174265, Pelitinib, Compound 56, XL647, PP3, AG-490, AG 555, Tyrphostin B42, Tyrphostin B44, AG 556, AG 494, AG 825, RG-13022, DAPH, Erbstatin Analog, JNJ 28871063, Tyrphostin 47, Lavendustin A, Lavendustin C, Lavendustin C methyl ester, LFM-A12, TAK 165, TAK 285, Tyrphostin 51, Tyrphostin AG 183, Tyrphostin AG 528, Tyrphostin AG 99, Tyrphostin RG 14620, WZ3146, WZ4002, WZ8040, Butein, Tyrphostin AG 112, etc.

The EGF receptor inhibitor is preferably AG1478. AG1478 is N-(3-chlorophenyl)-6,7-dimethoxy-4-quinazolin-4-amine, and has CAS number of 175178-82-2.

The EGF receptor inhibitor can be contained in a culture solution at a concentration of preferably 1 to 10 µM, more preferably 2 to 10 µM, and still more preferably 4 to 8 µM.

When a culture solution containing the EGF receptor inhibitor is used, proliferation of cells other than cardiomyocytes (e.g., fibroblasts) can be suppressed. When the proliferation of cells other than cardiomyocytes is suppressed, the ratio of the proliferated cardiomyocytes in the cell population (cardiomyocyte purity) can be increased. A mechanism by which the EGF receptor inhibitor suppresses the proliferation of cells other than cardiomyocytes, such as fibroblasts, is considered to be that a cell proliferation signal is suppressed by inhibition of an EGF signal or receptor tyrosine kinase. That is, although cells other than cardiomyocytes proliferate depending on a signal from a receptor tyrosine kinase such as an EGF receptor, since the proliferation of cardiomyocytes depends on a WNT signal, it is considered that the inhibition of the receptor tyrosine kinase suppresses only the proliferation of cells other than cardiomyocytes.

When a culture solution containing an EGF receptor inhibitor but not containing a WNT signal activator is used, a "cell population containing cardiomyocytes" is seeded at a cell density of preferably 2.0 × 10⁵ to 5.0 × 10⁵ cells/cm², and more preferably 2.0 × 10⁵ to 4.0 × 10⁵ cells/cm².

When a culture solution contains an EGF receptor inhibitor but does not contain a WNT signal activator as well, the culturing may be performed through a serial subculture. In the culturing of the second and subsequent passages, the same cell density as that in the previous culturing may be employed, or a cell density different from that in the previous culturing may be employed as long as the cell density is within a range of 0.1 × 10⁵ to 5.0 × 10⁵ cells/cm². In the culturing of the second and subsequent passages, the cardiomyocyte population obtained in the previous culturing is seeded at a cell density of preferably 2.0 × 10⁵ to 5.0 × 10⁵ cells/cm², and more preferably 2.0 × 10⁵ to 4.0 × 10⁵ cells/cm².

Thus, by combining "seeding of cells at an appropriate cell density" and "addition of an EGF receptor inhibitor to a culture solution", cardiomyocytes can be proliferated while suppressing proliferation of cells other than cardiomyocytes, whereby the cardiomyocyte purity can be increased. Therefore, according to this embodiment, a cardiomyocyte population having a high cardiomyocyte purity can be produced.

### <Preferred Embodiment: Embodiment Using WNT Signal Activator and EGF Receptor Inhibitor>

In the above method, the culture solution more preferably contains a WNT signal activator and an EGF receptor inhibitor. That is, in a more preferred embodiment, a method of producing a cardiomyocyte population includes culturing a cell population containing cardiomyocytes seeded at a cell density of 0.1 × 10⁵ to 5.0 × 10⁵ cells/cm² in a culture solution containing a WNT signal activator and an EGF receptor inhibitor to proliferate the cardiomyocytes.

More specifically, the method of producing a cardiomyocyte population can include
seeding a cell population containing cardiomyocytes in a culture vessel in a state of being suspended in a culture solution so as to have a cell density of 0.1 × 10⁵ to 5.0 × 10⁵ cells/cm², and
culturing the cell population in a culture solution containing a WNT signal activator and an EGF receptor inhibitor to proliferate the cardiomyocytes.

The WNT signal activator and the EGF receptor inhibitor may be added to the cell suspension before seeding or after seeding.

This embodiment can be performed by combining the "embodiment using a WNT signal activator" and the "embodiment using an EGF receptor inhibitor". For the WNT signal activator and the EGF receptor inhibitor, reference can be made to the above descriptions.

When a culture solution containing a WNT signal activator and an EGF receptor inhibitor is used, a "cell population containing cardiomyocytes" is seeded at a cell density of preferably 0.3 × 10⁵ to 4.0 × 10⁵ cells/cm², and more preferably 1.0 × 10⁵ to 3.0 × 10⁵ cells/cm².

When the culture solution contains a WNT signal activator and an EGF receptor inhibitor as well, the culturing may be performed through a serial subculture. In the culturing of the second and subsequent passages, the same cell density as that in the previous culturing may be employed, or a cell density different from that in the previous culturing may be employed as long as the cell density is within the range of 0.1 × 10⁵ to 5.0 × 10⁵ cells/cm². In the culturing of the second and subsequent passages, the cardiomyocyte population obtained in the previous culturing is seeded at a cell density of preferably 0.3 × 10⁵ to 4.0 × 10⁵ cells/cm², and more preferably 1.0 × 10⁵ to 3.0 × 10⁵ cells/cm².

Thus, by combining the "seeding of cells at an appropriate cell density" and the "addition of a WNT signal activator and an EGF receptor inhibitor to a culture solution", the proliferation rate of cardiomyocytes can be significantly increased while suppressing proliferation of cells other than cardiomyocytes. Therefore, according to this embodiment, a cardiomyocyte population having a high cardiomyocyte purity can be efficiently produced. In Example 3 to be described later, it is demonstrated that a cardiomyocyte population having a cardiomyocyte purity of 95% or more can be produced by performing culturing only once using a culture solution containing both a WNT signal activator and an EGF receptor inhibitor.

### (Effects)

Since the above-described method can be performed by seeding and culturing a cell population containing cardiomyocytes at a relatively high cell density, the method is simple and inexpensive. Further, in the above-described method, cardiomyocytes seeded at a relatively high cell density are cultured in a culture solution containing a WNT signal activator, whereby cardiomyocytes can be proliferated at a higher proliferation rate. According to the above-described method, a cardiomyocyte population having a high cardiomyocyte purity can be obtained by culturing cardiomyocytes seeded at a relatively high cell density in a culture solution containing an EGF receptor inhibitor.

Basically, almost no cases of neoplastic cardiomyocytes have been reported; therefore, a cardiomyocyte population with a high cardiomyocyte purity is considered to have a low risk of becoming a tumor and high safety. In addition, in a cardiomyocyte population having a high cardiomyocyte purity, variation in quality is less likely to occur between lots. Thus, a cardiomyocyte population having a high cardiomyocyte purity is excellent for practical use, and is particularly excellent as raw material cells for producing a transplantation material such as a cardiomyocyte sheet.

Conventionally, when proliferating and culturing cells, it has been common to ensure a space in which cells can proliferate and to seed the cells at a low cell density. On the other hand, in the above-described method, cardiomyocytes were able to be seeded in a state in which a space in which cells can proliferate is relatively small (i.e., at a relatively high cell density) and proliferated. In addition, in a conventionally common proliferation morphology of differentiated cells, it has been considered that when the cells proliferate in a culture vessel and reach a two-dimensionally confluent state, the cell proliferation stops or the proliferation rate significantly decreases. In contrast, in the above-described method, it was observed that cardiomyocytes three-dimensionally proliferate so as to rise in the Z-axis direction from the bottom surface of a culture vessel having no scaffold in a state of being adhered to the bottom surface even if there is no sufficient two-dimensional space in which the cardiomyocytes can proliferate. As described above, the present inventors consider that the above-described method is an invention which cannot be conceived from the conventional common technical knowledge in that a seeding density which is not used in the conventional proliferation culture is adopted and the differentiated cells are proliferated so as to rise on the bottom surface of the culture vessel.

### 2. Method of Proliferating Cardiomyocytes

According to another aspect, the method described in the above section "1. Method of Producing Cardiomyocyte Population" can also be referred to as a method of proliferating cardiomyocytes. That is, according to another aspect, there is provided a method of proliferating cardiomyocytes, the method including culturing a cell population containing cardiomyocytes seeded at a cell density of 0.1 × 10⁵ to 5.0 × 10⁵ cells/cm² in a culture solution to proliferate the cardiomyocytes. The method of proliferating cardiomyocytes can be performed according to the description in the above section "1. Method of Producing Cardiomyocyte Population".

### 3. Cardiomyocyte Population

When a cell population containing cardiomyocytes is cultured according to the method described in the above section "1. Method of Producing Cardiomyocyte Population", a cell population containing proliferated cardiomyocytes (hereinafter, also referred to as a cardiomyocyte population) can be obtained. Therefore, according to another aspect, there is provided a cardiomyocyte population produced by the method described in the above section "1. Method of Producing Cardiomyocyte Population".

### (Multinucleation of Cardiomyocytes)

When a cell population containing cardiomyocytes is cultured according to the method described in the above section "1. Method of Producing Cardiomyocyte Population", multinucleated cardiomyocytes may appear. Therefore, the cardiomyocyte population can contain multinucleated cardiomyocytes. Multinucleated cardiomyocytes with two to four nuclei are also found in cardiomyocytes in an actual human heart at a certain ratio. It is considered that a reason why the multinucleated cardiomyocytes appear is that mature cardiomyocytes increase a protein expression level and become larger. Cells before culturing (i.e., a cell population containing cardiomyocytes) do not substantially contain multinucleated cardiomyocytes, particularly multinucleated cardiomyocytes with three or more nuclei, whether they are prepared by inducing differentiation of pluripotent stem cells into cardiomyocytes or commercially obtained.

A multinucleated cardiomyocyte is a cardiomyocyte with two or more nuclei. The multinucleated cardiomyocyte can have, for example, up to eight nuclei. The multinucleated cardiomyocyte is, for example, a 2-nucleated cell, a 3-nucleated cell, a 4-nucleated cell, a 5-nucleated cell, a 6-nucleated cell, a 7-nucleated cell, or an 8-nucleated cell.

The multinucleated cardiomyocyte is particularly large and prominent compared to a mononucleated cardiomyocyte (i.e., a normal cardiomyocyte with one nucleus per cell). Specifically, the multinucleated cardiomyocytes can have an average maximum length of, for example, 50 to 1000 um when being measured in a state of being adhered to the bottom surface of the culture vessel. When a serial subculture is performed three times or more according to the method of the present invention, the multinucleated cardiomyocytes can have an average maximum length of, for example, 100 to 1000 µm when being measured in a state of being adhered to the bottom surface of the culture vessel. As used herein, the "average maximum length of multinucleated cardiomyocytes" refers to an average value of maximum lengths of five multinucleated cardiomyocytes. The "maximum length of multinucleated cardiomyocytes" can be measured by a two-dimensional image of cells observed under a microscope. In addition, when observed under a microscope, the multinucleated cardiomyocyte has a particularly large cell size, and in addition, a plurality of nuclei are present in a concentrated manner in one place, and the cytoplasm is particularly large. From these characteristics, the multinucleated cardiomyocytes can be observed under a microscope so as to be clearly distinguished from the mononucleated cardiomyocytes.

In the process of proliferation of cardiomyocytes by the method of the present invention, the ratio of multinucleated cardiomyocytes and the number of nuclei of multinucleated cardiomyocytes increase, and thus multinucleation of cardiomyocytes occurs with the proliferation. Therefore, when the number of passages of a serial subculture is increased, the ratio of multinucleated cardiomyocytes and the number of nuclei of multinucleated cardiomyocytes can be increased, and the size of multinucleated cardiomyocytes can be increased.

The cardiomyocyte population obtained after a first serial subculture can contain multinucleated cardiomyocytes with three or more nuclei at a ratio of 0.001 to 0.5%, for example, and can contain multinucleated cardiomyocytes with five or more nuclei at a ratio of 0.0001 to 0.05%, for example. After the first serial subculture, the multinucleated cardiomyocytes can have an average maximum length of, for example, 20 to 200 um. On the other hand, after the culturing of the second to fourth passages, the cardiomyocyte population can contain multinucleated cardiomyocytes with three or more nuclei at a ratio of 0.01 to 1%, for example, and can contain multinucleated cardiomyocytes with five or more nuclei at a ratio of 0.001 to 0.1%, for example. In addition, after the culturing of the second to fourth passages, the multinucleated cardiomyocytes can have an average maximum length of 50 to 1000 µm, for example.

As used herein, the term "ratio (%) of multinucleated cardiomyocytes" refers to a percentage of multinucleated cardiomyocytes in 10,000 cardiomyocytes.

The multinucleated cardiomyocytes, like mononucleated cardiomyocytes, are beating autonomously and function like mononucleated cardiomyocytes. In addition, as described above, it has been reported that the human heart contains multinucleated cardiomyocytes with two to four nuclei. Furthermore, compared to mononucleated cardiomyocytes, multinucleated cardiomyocytes are particularly large and have a large number of muscle fibers in the cytoplasm, and therefore have an advantage of strong muscle contractile force. Therefore, a cardiomyocyte population containing multinucleated cardiomyocytes is excellent as raw material cells for producing a transplantation material such as a cardiomyocyte sheet or cells for drug evaluation such as cardiotoxicity evaluation.

### (Maturation of Cardiomyocytes)

When a cell population containing cardiomyocytes is cultured according to the method described in the above section "1. Method of Producing Cardiomyocyte Population", the cardiomyocytes can be matured. Thus, the cardiomyocyte population contains mature cardiomyocytes. The mature cardiomyocytes can be identified by the cardiomyocytes having developed muscle fibers. The developed muscle fibers can be observed in a clear striped pattern when stained cardiomyocytes with antibodies against the structural protein (α-actinin) of the muscle sarcomere. When culturing is performed for a long period of time by increasing the number of passages of a serial subculture, the degree of maturity of cardiomyocytes can be further increased.

The mature cardiomyocytes are expected to reduce a risk of arrhythmias in transplantation and increase a therapeutic effect compared to non-mature cardiomyocytes. Therefore, a cardiomyocyte population containing mature cardiomyocytes is excellent as raw material cells for producing a transplantation material such as a cardiomyocyte sheet or cells for drug evaluation such as cardiotoxicity evaluation.

### (Increase in Cardiomyocyte Purity)

When a cell population containing cardiomyocytes is cultured according to the method described in the above section "1. Method of Producing Cardiomyocyte Population", the cardiomyocyte purity can be increased as described above. In particular, when a culture solution contains an EGF receptor inhibitor, the cardiomyocyte purity can be significantly increased. Thus, the cardiomyocyte population has a high cardiomyocyte purity. The cardiomyocyte population has a cardiomyocyte purity higher than that of the cell population before culturing, preferably 80% or more, more preferably 85% or more, still more preferably 90% or more, and still more preferably 95% or more. In addition, by increasing the number of passages of a serial subculture, the cardiomyocyte purity can be brought close to 100%.

As described above, since cardiomyocytes are cells that are unlikely to become a tumor, a cardiomyocyte population with a high cardiomyocyte purity has a low risk of becoming a tumor and high safety. In addition, in a cardiomyocyte population having a high cardiomyocyte purity, variation in quality is less likely to occur between lots. Thus, a cardiomyocyte population having a high cardiomyocyte purity is excellent for practical use, and is particularly excellent as raw material cells for producing a transplantation material such as a cardiomyocyte sheet or cells for drug evaluation such as cardiotoxicity evaluation.

### (Disappearance of Expression of Undifferentiated iPS Cell Marker Lin28)

The present inventors have newly found that when a cell population containing cardiomyocytes is cultured to produce a cardiomyocyte population according to the method described in the above section "1. Method of Producing Cardiomyocyte Population", the expression of undifferentiated iPS cell marker Lin28 disappears.

Lin28 is known to be an undifferentiated marker which is expressed in ES cells and iPS cells and whose expression is reduced in differentiated cells. Thus, Lin28 is used as a marker for detecting iPS cells remaining in differentiated cells produced by inducing differentiation of iPS cells. Since iPS cells have tumorigenicity, differentiated cells need to be used as a transplantation material after removing the iPS cells. A problem wherein iPS cells remain in differentiated cells is recognized as an important problem from a viewpoint of safety of transplantation materials.

Therefore, a "cardiomyocyte population not expressing Lin28" newly created by the present inventors hardly contains undifferentiated iPS cells, and thus is excellent in that it is unlikely to become a tumor.

### (Specific Embodiment)

In the following, a specific embodiment of a cardiomyocyte population will be described.

A cardiomyocyte population according to a first embodiment includes human multinucleated cardiomyocytes
having three or more nuclei and
derived from pluripotent stem cells.

Specifically, the cardiomyocyte population according to the first embodiment can include human multinucleated cardiomyocytes
having three to eight nuclei and
derived from pluripotent stem cells.

In the present specification, when it is expressed that "a cardiomyocyte population contains human multinucleated cardiomyocytes" without specifying a ratio of human multinucleated cardiomyocytes in the cardiomyocyte population, this expression means that it suffices that the cardiomyocyte population contains at least one human multinucleated cardiomyocyte.

The "human multinucleated cardiomyocytes derived from pluripotent stem cells" can refer to multinucleated cardiomyocytes obtained by inducing differentiation of human pluripotent stem cells (e.g., human iPS cells) into cardiomyocytes and culturing the obtained cardiomyocytes according to the method described in the section "1. Method of Producing Cardiomyocyte Population".

In the first embodiment, the cardiomyocyte population has a cardiomyocyte purity of preferably 80% or more, more preferably 85% or more, still more preferably 90% or more, and still more preferably 95% or more. In the first embodiment, the human multinucleated cardiomyocytes have an average maximum length of, for example, 50 to 1000 pm, and preferably 50 to 500 pm, when measured in a state of being adhered to the bottom surface of the culture vessel. In the first embodiment, the human multinucleated cardiomyocytes preferably have developed muscle fibers. In the first embodiment, the cardiomyocyte population can contain the "human multinucleated cardiomyocytes having three or more nuclei and derived from pluripotent stem cells" at a discretionary ratio. For example, when a cardiomyocyte population is obtained according to the method described in the section "1. Method of Producing Cardiomyocyte Population", the cardiomyocyte population can contain the "human multinucleated cardiomyocytes having three or more nuclei and derived from pluripotent stem cells" at a ratio of, for example, 0.0001 to 1%. The ratio of "human multinucleated cardiomyocytes having three or more nuclei and derived from pluripotent stem cells" in the cardiomyocyte population can be made 100% by sorting and collecting only multinucleated cells.

A cardiomyocyte population according to a second embodiment contains human multinucleated cardiomyocytes with five or more nuclei. Specifically, the cardiomyocyte population according to the second embodiment can contain human multinucleated cardiomyocytes with five to eight nuclei.

In the second embodiment, the cardiomyocyte population has a cardiomyocyte purity of preferably 80% or more, more preferably 85% or more, still more preferably 90% or more, and still more preferably 95% or more. In the second embodiment, the human multinucleated cardiomyocytes have an average maximum length of, for example, 50 to 1000 pm, and preferably 50 to 500 pm, when measured in a state of being adhered to the bottom surface of the culture vessel. In the second embodiment, the human multinucleated cardiomyocytes preferably have developed muscle fibers. In the second embodiment, the cardiomyocyte population can contain the "human multinucleated cardiomyocytes with five or more nuclei" at a discretionary ratio. For example, when a cardiomyocyte population is obtained according to the method described in the section "1. Method of Producing Cardiomyocyte Population", the cardiomyocyte population can contain the "human multinucleated cardiomyocytes with five or more nuclei" at a ratio of, for example, 0.0001 to 1%. The ratio of "human multinucleated cardiomyocytes with five or more nuclei" in the cardiomyocyte population can be increased to 100% by sorting and collecting only multinucleated cells.

### 4. Transplantation Material

The cardiomyocyte population described in the above section of "3. Cardiomyocyte Population" can be used as raw material cells for producing a transplantation material. Examples of the transplantation material include a cardiomyocyte sheet, a cardiomyocyte population for a single cell infusion transplantation, etc. Therefore, according to another aspect, there is provided a transplantation material including a cardiomyocyte population produced by the method described in the above section "1. Method of Producing Cardiomyocyte Population".

Specific embodiments of the transplantation material will be described below.

A transplantation material according to a first embodiment includes a cardiomyocyte population containing human multinucleated cardiomyocytes
having three or more nuclei and
derived from pluripotent stem cells.

Specifically, the transplantation material according to the first embodiment can include a cardiomyocyte population containing human multinucleated cardiomyocytes having three to eight nuclei and
derived from pluripotent stem cells.

In the present specification, when it is expressed that "a transplantation material contains human multinucleated cardiomyocytes" without specifying a ratio of human multinucleated cardiomyocytes in the transplantation material, this expression means that it suffices that the transplantation material contains at least one human multinucleated cardiomyocyte.

In the first embodiment, the cardiomyocyte population has a cardiomyocyte purity of preferably 80% or more, more preferably 85% or more, still more preferably 90% or more, and still more preferably 95% or more. In the first embodiment, the human multinucleated cardiomyocytes have an average maximum length of, for example, 50 to 1000 pm, and preferably 50 to 500 pm, when measured in a state of being adhered to the bottom surface of the culture vessel. In the first embodiment, the human multinucleated cardiomyocytes preferably have developed muscle fibers. In the first embodiment, the cardiomyocyte population can contain the "human multinucleated cardiomyocytes having three or more nuclei and derived from pluripotent stem cells" at a discretionary ratio. For example, when a cardiomyocyte population is obtained according to the method described in the section "1. Method of Producing Cardiomyocyte Population", the cardiomyocyte population can contain the "human multinucleated cardiomyocytes having three or more nuclei and derived from pluripotent stem cells" at a ratio of, for example, 0.0001 to 1%. The ratio of "human multinucleated cardiomyocytes having three or more nuclei and derived from pluripotent stem cells" in the cardiomyocyte population can be made 100% by sorting and collecting only multinucleated cells.

A transplantation material according to a second embodiment includes a cardiomyocyte population containing human multinucleated cardiomyocytes with five or more nuclei. Specifically, the transplantation material according to the second embodiment can include a cardiomyocyte population containing human multinucleated cardiomyocytes with five to eight nuclei.

In the second embodiment, the cardiomyocyte population has a cardiomyocyte purity of preferably 80% or more, more preferably 85% or more, still more preferably 90% or more, and still more preferably 95% or more. In the second embodiment, the human multinucleated cardiomyocytes have an average maximum length of, for example, 50 to 1000 pm, and preferably 50 to 500 pm, when measured in a state of being adhered to the bottom surface of the culture vessel. In the second embodiment, the human multinucleated cardiomyocytes preferably have developed muscle fibers. In the second embodiment, the cardiomyocyte population can contain the human multinucleated cardiomyocytes with five or more nuclei at a discretionary ratio. For example, when a cardiomyocyte population is obtained according to the method described in the section "1. Method of Producing Cardiomyocyte Population", the cardiomyocyte population can contain the "human multinucleated cardiomyocytes with five or more nuclei" at a ratio of, for example, 0.0001 to 1%. The ratio of "human multinucleated cardiomyocytes with five or more nuclei" in the cardiomyocyte population can be increased to 100% by sorting and collecting only multinucleated cells.

### 5. Multinucleated Cardiomyocytes

As described in the above section "3. Cardiomyocyte Population", the cardiomyocyte population has an increased number of multinucleated cardiomyocytes compared to the cells before culturing (cell population containing cardiomyocytes). Therefore, according to another aspect, there are provided human multinucleated cardiomyocytes with five or more nuclei. Specifically, human multinucleated cardiomyocytes with five to eight nuclei can be provided.

### 6. Kit for Proliferating Cardiomyocytes

According to another aspect, there is provided a kit for proliferating cardiomyocytes, including a cell population containing cardiomyocytes, a culture solution for cardiomyocytes, a WNT signal activator, and an EGF receptor inhibitor. Here, for the "cell population containing cardiomyocytes", the "cell population containing cardiomyocytes" described in the section "1. Method of Producing Cardiomyocyte Population" can be referred to, for the "culture solution for cardiomyocytes", the "culture solution" described in the section "1. Method of Producing Cardiomyocyte Population" can be referred to, for the "WNT signal activator", the "WNT signal activator" described in the section "1. Method of Producing Cardiomyocyte Population" can be referred to, and for the "EGF receptor inhibitor", the "EGF receptor inhibitor" described in the section "1. Method of Producing Cardiomyocyte Population" can be referred to.

The kit may further include a culture vessel. For the "culture vessel", the "culture vessel" described in the section "1. Method of Producing Cardiomyocyte Population" can be referred to.

The kit may further include an instruction manual describing the method of seeding and the method of culturing. In the instruction manual, it is possible to explain the method of seeding and the method of culturing, for example, a seeding density of a cell population containing cardiomyocytes, an amount of a culture solution to be filled in a culture vessel, a concentration of a WNT signal activator to be added, a concentration of an EGF receptor inhibitor to be added, etc.

When a cell population containing cardiomyocytes is cultured using the kit for proliferating cardiomyocytes, the cardiomyocytes can be proliferated.

### 7. Preferred Embodiments

Preferred embodiments of the present invention will be summarized below.
[A1] A method of producing a cardiomyocyte population, the method including culturing a cell population containing cardiomyocytes seeded at a cell density of 0.1 × 10⁵ to 5.0 × 10⁵ cells/cm² in a culture solution to proliferate the cardiomyocytes.
[A2] A method of producing a cardiomyocyte population, the method including seeding a cell population containing cardiomyocytes at a cell density of 0.1 × 10⁵ to 5.0 × 10⁵ cells/cm², and
   culturing the seeded cell population in a culture solution to proliferate the cardiomyocytes.
[A3] The method according to [A1] or [A2], wherein the cell density is 2.0 × 10⁵ to 5.0 × 10⁵ cells/cm², and preferably 2.0 × 10⁵ to 4.0 × 10⁵ cells/cm².
[A4] The method according to any one of [A1] to [A3], wherein the cell population containing cardiomyocytes is a cell population containing human cardiomyocytes.
[A5] The method according to any one of [A1] to [A4], wherein the cell population containing cardiomyocytes is a cell population obtained by inducing differentiation of pluripotent stem cells into cardiomyocytes, preferably a cell population obtained by inducing differentiation of iPS cells into cardiomyocytes, and more preferably a cell population obtained by inducing differentiation of human iPS cells into cardiomyocytes.
[A6] The method according to any one of [A1] to [A5], wherein the cell population has a cardiomyocyte purity of 80 to 100%, and preferably 90 to 100%, at a start of the culturing.
[A7] The method according to any one of [A1] to [A6], wherein the cell population containing cardiomyocytes is seeded in a single-cell state.
[A8] The method according to any one of [A1] to [A7], wherein the culturing is performed under a condition in which a distance from a liquid surface of the culture solution to a culture vessel bottom surface in contact with the cardiomyocytes is 3 to 30 mm, and preferably 5 to 10 mm.
[A9] The method according to any one of [A1] to [A8], wherein the culture solution contains serum.
[A10] The method according to any one of [A1] to [A9], wherein the culturing is performed through a serial subculture.
[A11] The method according to any one of [A1] to [A10], further comprising seeding at least a part of the cardiomyocyte population obtained through the culturing at a cell density of 0.1 × 10⁵ to 5.0 × 10⁵ cells/cm² and performing a serial subculture.
[A12] The method according to any one of [A1] to [A11], further comprising seeding at least a part of the cardiomyocyte population obtained through the culturing at a cell density of 0.1 × 10⁵ to 5.0 × 10⁵ cells/cm², and performing a serial subculture in a culture solution having the same composition as in the culturing.
[A13] The method according to any one of [A1] to [A12], wherein the culturing is performed through an adhesion culture.
[B1] The method according to any one of [A1] to [A13], wherein the culture solution contains a WNT signal activator.
[B2] The method according to [B1], wherein the cell density is 0.3 × 10⁵ to 4.0 × 10⁵ cells/cm², and preferably 1.0 × 10⁵ to 3.0 × 10⁵ cells/cm².
[B3] The method according to [B1] or [B2], wherein the WNT signal activator is CHIR99021.
[B4] The method according to any one of [B1] to [B3], wherein the WNT signal activator is contained in the culture solution at a concentration of 1 to 5 µM, preferably 2 to 5 µM, and more preferably 2 to 4 µM.
[C1] The method according to any one of [A1] to [A13], wherein the culture solution contains an EGF receptor inhibitor.
[C2] The method according to [C1], wherein the cell density is 2.0 × 10⁵ to 5.0 × 10⁵ cells/cm², and preferably 2.0 × 10⁵ to 4.0 × 10⁵ cells/cm².
[C3] The method according to [C1] or [C2], wherein the EGF receptor inhibitor is AG1478.
[C4] The method according to any one of [C1] to [C3], wherein the EGF receptor inhibitor is contained in the culture solution at a concentration of 1 to 10 µM, preferably 2 to 10 µM, and more preferably 4 to 8 µM.
[D1] The method according to any one of [A1] to [A13], wherein the culture solution contains a WNT signal activator and an EGF receptor inhibitor.
[D2] The method according to [D1], wherein the cell density is 0.3 × 10⁵ to 4.0 × 10⁵ cells/cm², and preferably 1.0 × 10⁵ to 3.0 × 10⁵ cells/cm².
[D3] The method according to [D1] or [D2], wherein the WNT signal activator is CHIR99021 and the EGF receptor inhibitor is AG1478.
[D4] The method according to any one of [D1] to [D3], wherein the WNT signal activator is contained in the culture solution at a concentration of 1 to 5 µM, preferably 2 to 5 µM, and more preferably 2 to 4 µM, and the EGF receptor inhibitor is contained in the culture solution at a concentration of 1 to 10 µM, preferably 2 to 10 µM, and more preferably 4 to 8 µM.
[E1] A cardiomyocyte population containing human multinucleated cardiomyocytes having three or more nuclei and derived from pluripotent stem cells.
[E2] A cardiomyocyte population containing human multinucleated cardiomyocytes having three to eight nuclei and derived from pluripotent stem cells.
[E3] The cardiomyocyte population according to [E1] or [E2], wherein the cardiomyocyte population has a cardiomyocyte purity of 80% or more, preferably 85% or more, more preferably 90% or more, and still more preferably 95% or more.
[E4] The cardiomyocyte population according to any one of [E1] to [E3], wherein the human multinucleated cardiomyocytes have an average maximum length of 50 to 1000 pm, and preferably 50 to 500 pm, when measured in a state of being adhered to a bottom surface of a culture vessel.
[E5] The cardiomyocyte population according to any one of [E1] to [E4], wherein the human multinucleated cardiomyocytes have developed muscle fibers.
[F1] A transplantation material including a cardiomyocyte population containing human multinucleated cardiomyocytes having three or more nuclei and derived from pluripotent stem cells.
[F2] A transplantation material including a cardiomyocyte population containing human multinucleated cardiomyocytes having three to eight nuclei and derived from pluripotent stem cells.
[F3] The transplantation material according to [F1] or [F2], wherein the cardiomyocyte population has a cardiomyocyte purity of 80% or more, preferably 85% or more, more preferably 90% or more, and still more preferably 95% or more.
[F4] The transplantation material according to any one of [F1] to [F3], wherein the human multinucleated cardiomyocytes have an average maximum length of 50 to 1000 pm, and preferably 50 to 500 pm, when measured in a state of being adhered to a bottom surface of a culture vessel.
[F5] The transplantation material according to any one of [F1] to [F4], wherein the human multinucleated cardiomyocytes have developed muscle fibers.
[G1] Human multinucleated cardiomyocytes with five or more nuclei.
[H1] A cardiomyocyte population containing human multinucleated cardiomyocytes with five or more nuclei.
[H2] A cardiomyocyte population containing human multinucleated cardiomyocytes with five to eight nuclei.
[H3] A cardiomyocyte population containing human multinucleated cardiomyocytes having five or more nuclei and derived from pluripotent stem cells.
[H4] A cardiomyocyte population containing human multinucleated cardiomyocytes having five to eight nuclei and derived from pluripotent stem cells.
[H5] The cardiomyocyte population according to any one of [H1] to [H4], wherein the cardiomyocyte population has a cardiomyocyte purity of 80% or more, preferably 85% or more, more preferably 90% or more, and still more preferably 95% or more.
[H6] The cardiomyocyte population according to any one of [H1] to [H5], wherein the human multinucleated cardiomyocytes have an average maximum length of 50 to 1000 pm, and preferably 50 to 500 pm, when measured in a state of being adhered to a bottom surface of a culture vessel.
[H7] The cardiomyocyte population according to any one of [H1] to [E6], wherein the human multinucleated cardiomyocytes have developed muscle fibers.
[I1] A transplantation material including a cardiomyocyte population containing human multinucleated cardiomyocytes with five or more nuclei.
[I2] A transplantation material including a cardiomyocyte population containing human multinucleated cardiomyocytes with five to eight nuclei.
[I3] A transplantation material including a cardiomyocyte population containing human multinucleated cardiomyocytes having five or more nuclei and derived from pluripotent stem cells.
[I4] A transplantation material including a cardiomyocyte population containing human multinucleated cardiomyocytes having five to eight nuclei and derived from pluripotent stem cells.
[I5] The transplantation material according to any one of [I1] to [I4], wherein the cardiomyocyte population has a cardiomyocyte purity of 80% or more, preferably 85% or more, more preferably 90% or more, and still more preferably 95% or more.
[I6] The transplantation material according to any one of [I1] to [I5], wherein the human multinucleated cardiomyocytes have an average maximum length of 50 to 1000 pm, and preferably 50 to 500 pm, when measured in a state of being adhered to a bottom surface of a culture vessel.
[I7] The transplantation material according to any one of [I1] to [I6], wherein the human multinucleated cardiomyocytes have developed muscle fibers.
[J1] A cardiomyocyte population produced by the method according to any one of [A1] to [A13], [B1] to [B4], [C1] to [C4], and [D1] to [D4].
[J2] The cardiomyocyte population according to [J1], wherein the cardiomyocyte population includes multinucleated cardiomyocytes (e.g., cardiomyocytes with two to eight nuclei), preferably cardiomyocytes with three or more nuclei (e.g., cardiomyocytes with three to eight nuclei), and more preferably cardiomyocytes with five or more nuclei (e.g., cardiomyocytes with five to eight nuclei).
[J3] The cardiomyocyte population according to [J1] or [J2], wherein the cardiomyocyte population has a cardiomyocyte purity of 80% or more, preferably 85% or more, more preferably 90% or more, and still more preferably 95% or more.
[J4] The cardiomyocyte population according to any one of [J1] to [J3], wherein the multinucleated cardiomyocytes have an average maximum length of 50 to 1000 µm, and preferably 50 to 500 µm, when measured in a state of being adhered to a bottom surface of a culture vessel.
[J5] The cardiomyocyte population according to any one of [J1] to [J4], wherein the multinucleated cardiomyocytes have developed muscle fibers.
[K1] A transplantation material containing a cardiomyocyte population produced by the method according to any one of [A1] to [A13], [B1] to [B4], [C1] to [C4], and [D1] to [D4].
[K2] The transplantation material according to [K1], wherein the cardiomyocyte population includes multinucleated cardiomyocytes (e.g., cardiomyocytes with two to eight nuclei), preferably cardiomyocytes with three or more nuclei (e.g., cardiomyocytes with three to eight nuclei), and more preferably cardiomyocytes with five or more nuclei (e.g., cardiomyocytes with five to eight nuclei).
[K3] The transplantation material according to [K1] or [K2], wherein the cardiomyocyte population has a cardiomyocyte purity of 80% or more, preferably 85% or more, more preferably 90% or more, and still more preferably 95% or more.
[K4] The transplantation material according to any one of [K1] to [K3], wherein the multinucleated cardiomyocytes have an average maximum length of 50 to 1000 µm, and preferably 50 to 500 µm, when measured in a state of being adhered to a bottom surface of a culture vessel.
[K5] The transplantation material according to any one of [K1] to [K4], wherein the multinucleated cardiomyocytes have developed muscle fibers.
[L1] A kit for proliferating cardiomyocytes, the kit including a cell population containing cardiomyocytes, a culture solution for cardiomyocytes, a WNT signal activator, and an EGF receptor inhibitor.
[L2] The kit according to [L1], wherein the cell population containing cardiomyocytes is a cell population containing human cardiomyocytes.
[L3] The kit according to [L1] or [L2], wherein the cell population containing cardiomyocytes is a cell population obtained by inducing differentiation of pluripotent stem cells into cardiomyocytes, preferably a cell population obtained by inducing differentiation of iPS cells into cardiomyocytes, and more preferably a cell population obtained by inducing differentiation of human iPS cells into cardiomyocytes.
[L4] The kit according to any one of [L1] to [L3], wherein the cell population containing cardiomyocytes has a cardiomyocyte purity of 80 to 100%, and preferably 90 to 100%.
[L5] The kit according to any one of [L1] to [L4], further including a culture vessel.
[L6] The kit according to any one of [L1] to [L5], further including an instruction manual describing how to seed the cell population and how to culture the cell population.

### [Examples]

### [Example 1]

### [1-1] Method

Differentiation induction of human iPS cells (253G1 strain, Riken BioResource Center) was performed using a protein-free cardiomyocyte differentiation induction method, and cardiomyocytes (cTnT positive rate: 90 to 96%) from the 37th day from a start of differentiation induction were prepared. The cardiomyocytes were dispersed with trypsin (Life Technology) and then suspended in a 20% FBS cardiomyocyte differentiation culture medium (containing 3 µM Y-27632) in a state of single cardiomyocytes. The cardiomyocyte differentiation culture medium is commercially available from Fujifilm Wako Pure Chemical Industries, Ltd., and its composition is composed of 245 ml of IMDM, 245 ml of DMDM, 5 ml of MEM non-essential amino acid solution (× 100), 5 ml of 0.2 M L-glutamine, 100 ul of 1M L-carnitine, 50 mg of ascorbate, and 1 ml of 0.5 M creatine. The suspended cardiomyocytes were seeded in T25 flasks (Iwaki) coated with iMatrix laminin511 (Nippi, Incorporated).

The cardiomyocytes were seeded at the following cell densities.
1.0 × 10⁶ cells/T25 flask (i.e., 0.4 × 10⁵ cells/cm²)
2.5 × 10⁶ cells/T25 flask (i.e., 1.0 × 10⁵ cells/cm²)
5.0 × 10⁶ cells/T25 flask (i.e., 2.0 × 10⁵ cells/cm²)
8.0 × 10⁶ cells/T25 flask (i.e., 3.2 × 10⁵ cells/cm²)

A cell-containing culture solution was placed in the culture vessel so that a distance from a liquid surface of the culture solution to a bottom surface of the culture vessel was 10 mm.

On the day after the seeding (day 1), the culture medium was replaced with a 2% FBS cardiomyocyte differentiation culture medium, and thereafter the culture medium was replaced every 3 to 4 days and culturing was performed for 2 weeks. After two weeks of the culturing, the following analysis was performed.

### [1-2] Analysis

The cells obtained after the culturing were dispersed using trypsin EDTA, the number of cells was counted, the cells were treated with a cardiac troponin T (cTnT) antibody, and the ratio of cardiomyocytes (cTnT positive rate) was examined by flow cytometry. The ratio of cardiomyocytes (cTnT positive rate) represents a "cardiomyocyte purity". A "cardiomyocyte proliferation rate" was determined by dividing the number of cardiomyocytes after the culturing by the number of cardiomyocytes before the culturing.

A "ratio of multinucleated cardiomyocytes" was determined from a percentage of multinucleated cardiomyocytes in 10,000 cardiomyocytes. An "average maximum length of multinucleated cardiomyocytes" was determined by measuring the maximum lengths of five multinucleated cardiomyocytes from a two-dimensional microscopic image and calculating an average of the measured values.

### [1-3] Result

### (a) Results of Cardiomyocyte Proliferation Rate and Cardiomyocyte Purity

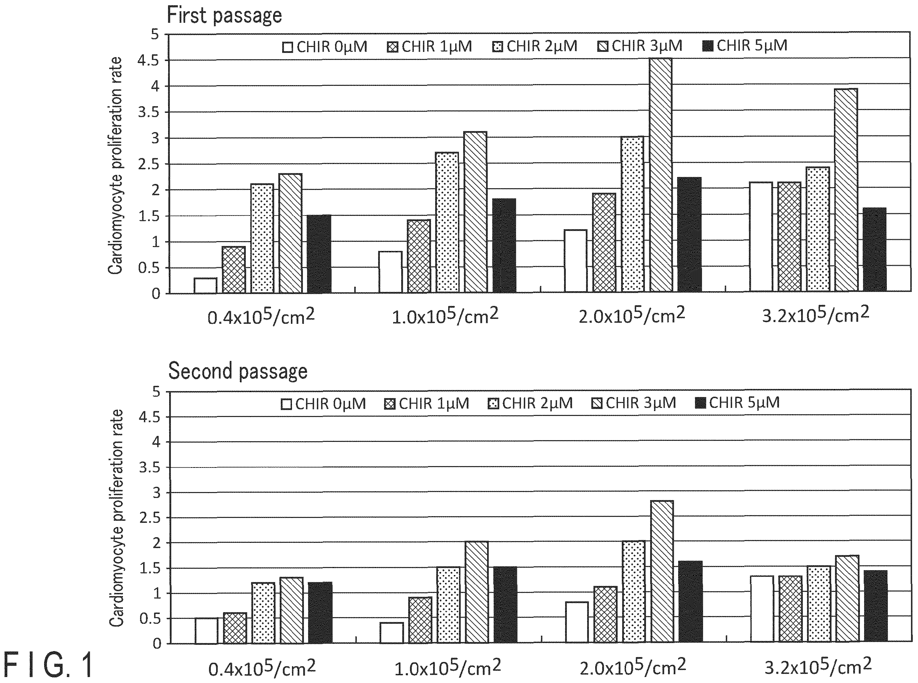

When the cells were cultured at the seeding density of 2.0 × 10⁵ cells/cm², the number of cardiomyocytes after 2 weeks of culturing was 6 × 10⁶ cells/T25 flask, and the cardiomyocytes proliferated 1.2-fold (see the upper graph in FIG. 1). In this case, the cTnT positive rate (cardiomyocyte purity) after 2 weeks of culturing was 92%, which was almost the same as the cardiomyocyte purity before the culturing.

When the cells were cultured at the seeding density of 3.2 × 10⁵ cells/cm², the number of cardiomyocytes after 2 weeks of culturing was 17 × 10⁶ cells/T25 flask, and the cardiomyocytes proliferated 2.1-fold (see the upper graph in FIG. 1). In this case, the cTnT positive rate (cardiomyocyte purity) after 2 weeks of culturing was 92%, which was almost the same as the cardiomyocyte purity before the culturing.

From these results, it is found that when a cell population containing cardiomyocytes is cultured at an appropriate seeding density (preferably 2.0 × 10⁵ to 5.0 × 10⁵ cells/cm², and more preferably 2.0 × 10⁵ to 4.0 × 10⁵ cells/cm²), the cardiomyocytes can be proliferated. Higher seeding density tended to increase the cardiomyocyte proliferation rate (see FIG. 1).

### (b) Result of Cardiomyocyte Population

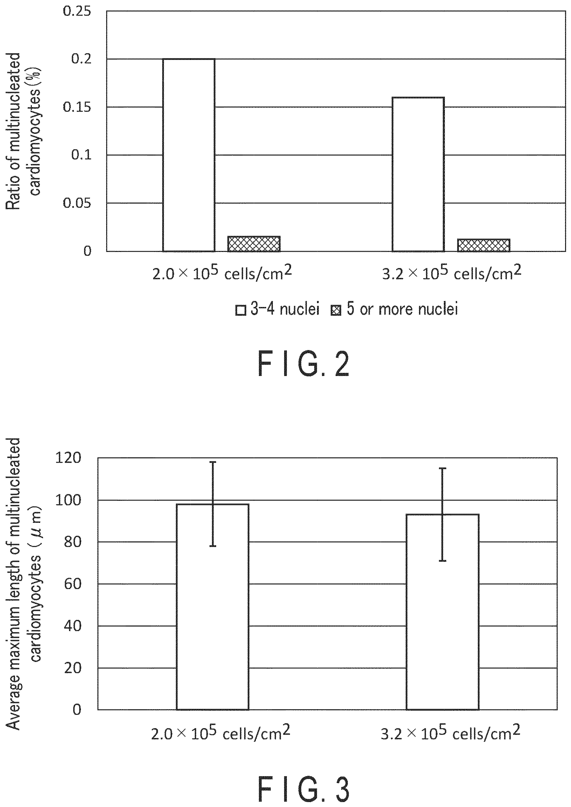

A ratio of multinucleated cardiomyocytes is shown in FIG. 2, and an average maximum length of the multinucleated cardiomyocytes is shown in FIG. 3.

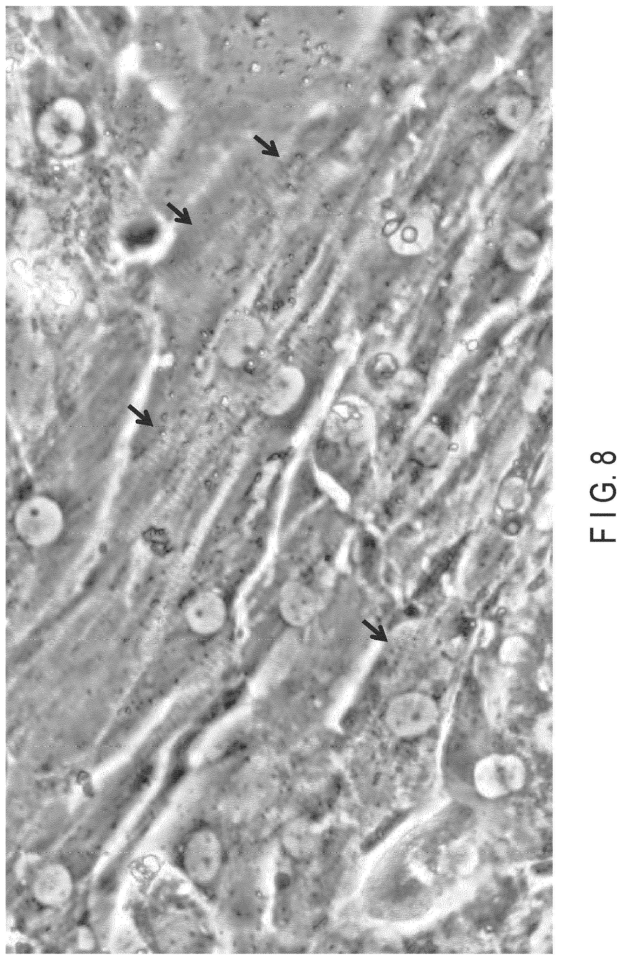

When cultured at the seeding density of 2.0 × 10⁵ cells/cm², the obtained cardiomyocyte population contained multinucleated cardiomyocytes with three or four nuclei at a ratio of 0.2% and multinucleated cardiomyocytes with five or more nuclei at a ratio of 0.015% (see FIG. 2). That is, the obtained cardiomyocyte population contained multinucleated cardiomyocytes with three or more nuclei at a ratio of 0.215%. In this case, multinucleated cardiomyocytes (i.e. cardiomyocytes with two or more nuclei) had an average maximum length of 98 um (see FIG. 3). In addition, striped sarcomeric structures were observed in the multinucleated cardiomyocytes (see FIG. 8).

When cultured at the seeding density of 3.2 × 10⁵ cells/cm², the cardiomyocyte population contained multinucleated cardiomyocytes with three or four nuclei at a ratio of 0.16% and multinucleated cardiomyocytes with five or more nuclei at a ratio of 0.012% (see FIG. 2). That is, the obtained cardiomyocyte population contained multinucleated cardiomyocytes with three or more nuclei at a ratio of 0.172%. In this case, multinucleated cardiomyocytes (i.e., cardiomyocytes with two or more nuclei) had an average maximum length of 93 um (see FIG. 3). In addition, striped sarcomeric structures were observed in the multinucleated cardiomyocytes (see FIG. 8).

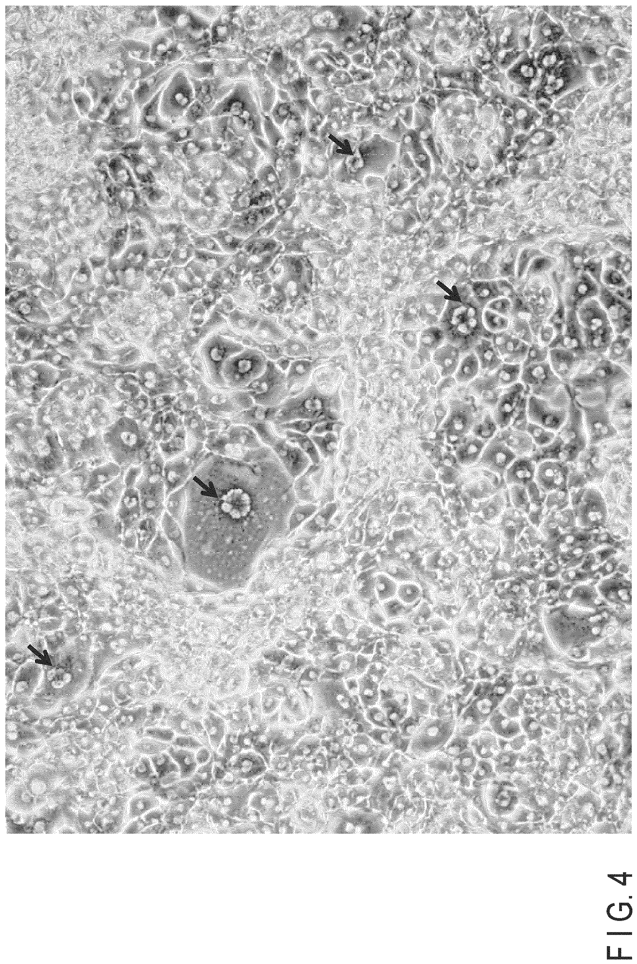

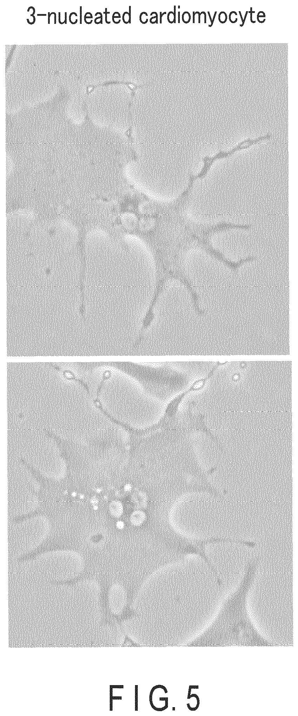

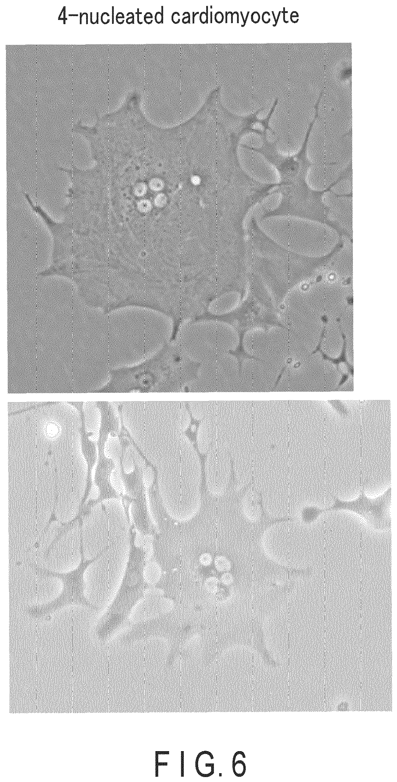

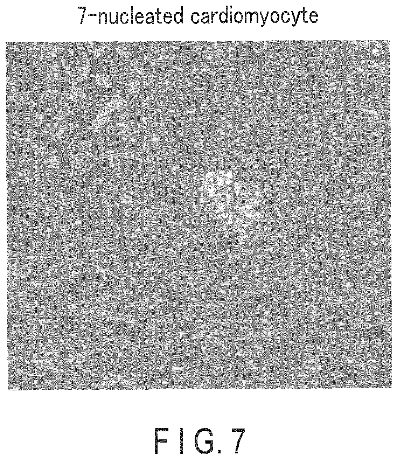

An example of the cardiomyocyte population obtained in Example 1 is shown in FIG. 4. In FIG. 4, multinucleated cardiomyocytes are indicated by arrows. The multinucleated cardiomyocytes were large in size and prominent in the cardiomyocyte population. Examples of the multinucleated cardiomyocytes contained in the cardiomyocyte population are shown in FIGS. 5 to 7. FIG. 5 shows an example of a 3-nucleated cardiomyocyte, FIG. 6 shows an example of a 4-nucleated cardiomyocyte, and FIG. 7 shows an example of a 7-nucleated cardiomyocyte. FIG. 8 shows the sarcomere structures in the multinucleated cardiomyocytes. In FIG. 8, the sarcomeric structures are indicated by arrows.

### [Example 2]

In Example 2, an effect of addition of a WNT signal activator (CHIR99021) was examined. CHIR99021 was added to the culture solution at a final concentration of 1 µM, 2 µM, 3 µM, or 5 µM.

### [2-1] Method

Differentiation induction of human iPS cells (253G1 strain, Riken BioResource Center) was performed using a protein-free cardiomyocyte differentiation induction method, and cardiomyocytes (cTnT positive rate: 90 to 96%) from the 37th day from a start of differentiation induction were prepared. The cardiomyocytes were dispersed with trypsin (Life Technology), and then suspended in a 20% FBS cardiomyocyte differentiation culture medium (containing 3 µM Y-27632 and 1 to 5 µM CHIR99021) in a state of single cardiomyocytes. The suspended cardiomyocytes were seeded in T25 flasks (Iwaki) coated with iMatrix laminin511 (Nippi, Incorporated).

The cardiomyocytes were seeded at the following cell densities.
1.0 × 10⁶ cells/T25 flask (i.e., 0.4 × 10⁵ cells/cm²)
2.5 × 10⁶ cells/T25 flask (i.e., 1.0 × 10⁵ cells/cm²)
5.0 × 10⁶ cells/T25 flask (i.e., 2.0 × 10⁵ cells/cm²)
8.0 × 10⁶ cells/T25 flask (i.e., 3.2 × 10⁵ cells/cm²)

The cell-containing culture solution was placed in the culture vessel so that a distance from a liquid surface of the culture solution to a bottom surface of the culture vessel was 7 mm.

On the day after the seeding (day 1), the culture medium was replaced with a 2% FBS cardiomyocyte differentiation culture medium (containing 1 to 5 µM CHIR99021). Thereafter, the culture medium was replaced every 3 to 4 days, and culturing was performed for 2 weeks (a first serial subculture). After the 2 weeks of culturing, the cells were dispersed using trypsin-EDTA, and the obtained single cardiomyocytes were seeded in a flask at the same cell density as in the previous culturing and a serial subculture was performed under the same condition as described above (a second serial subculture).

When the seeding density of the cells was 2.0 × 10⁵ cells/cm² and the concentration of CHIR99021 was 3 µM, the cells were cultured to a fourth serial subculture.

### [2-2] Analysis

For the cells obtained after each serial subculture, "cardiomyocyte purity", "cardiomyocyte proliferation rate", "ratio of multinucleated cardiomyocytes", and "average maximum length of multinucleated cardiomyocytes" were determined according to the same procedure as in Example 1.

### [2-3] Result

### (a) Results of Cardiomyocyte Proliferation Rate and Cardiomyocyte Purity

Cardiomyocyte proliferation rates in the first serial subculture are shown in the upper graph of FIG. 1. Cardiomyocyte proliferation rates in the second serial subculture are shown in the lower graph of FIG. 1.

In the first serial subculture, when the cell population containing cardiomyocytes was seeded at appropriate cell densities (preferably 0.3 × 10⁵ to 4.0 × 10⁵ cells/cm², and more preferably 1.0 × 10⁵ to 3.0 × 10⁵ cells/cm²) and cultured by adding CHIR99021 (1 to 5 µM) to the culture solutions, higher proliferation rates could be achieved as compared with the case in which CHIR99021 was not added (see the upper graph in FIG. 1). When CHIR99021 was added, the cTnT positive rate (cardiomyocyte purity) after the first serial subculture was 90% or more, which was almost the same as the cardiomyocyte purity before the culturing.

When the second serial subculture was performed, the proliferation rate tended to decrease compared to the first serial subculture. However, when CHIR99021 (1 to 5 µM) was added to the culture solutions, higher proliferation rates were obtained (see the lower graph in FIG. 1). When CHIR99021 was added, the cTnT positive rate (cardiomyocyte purity) after the second serial subculture was 90% or more, which was almost the same as the cardiomyocyte purity before the culturing.

In both the first serial subculture and the second serial subculture, when the concentration of CHIR99021 was 3 µM, the cardiomyocyte proliferation rate was the highest. When the seeding density of the cells was 2.0 × 10⁵ cells/cm² and the concentration of CHIR99021 was 3 µM, the cardiomyocytes proliferated 4.5-fold in the first serial subculture and 2.8-fold in the second serial subculture. That is, after the second serial subculture, the cardiomyocytes proliferated 12.6-fold in total. In addition, when the seeding density of the cells was 2.0 × 10⁵ cells/cm² and the concentration of CHIR99021 was 3 µM, the cardiomyocytes proliferated 22.7-fold in total after the third to fourth serial subcultures.

From the above results, it can be seen that when a cell population containing cardiomyocytes is seeded at an appropriate cell density (preferably 0.3 × 10⁵ to 4.0 × 10⁵ cells/cm², more preferably 1.0 × 10⁵ to 3.0 × 10⁵ cells/cm², and most preferably 2.0 × 10⁵ cells/cm²) and cultured by adding a WNT signal activator at an appropriate concentration (preferably 1 to 5 µM, more preferably 2 to 5 µM, still more preferably 2 to 4 µM, and most preferably 3 µM) to the culture solution, the cardiomyocytes can be more efficiently proliferated.

### (b) Result of Cardiomyocyte Population

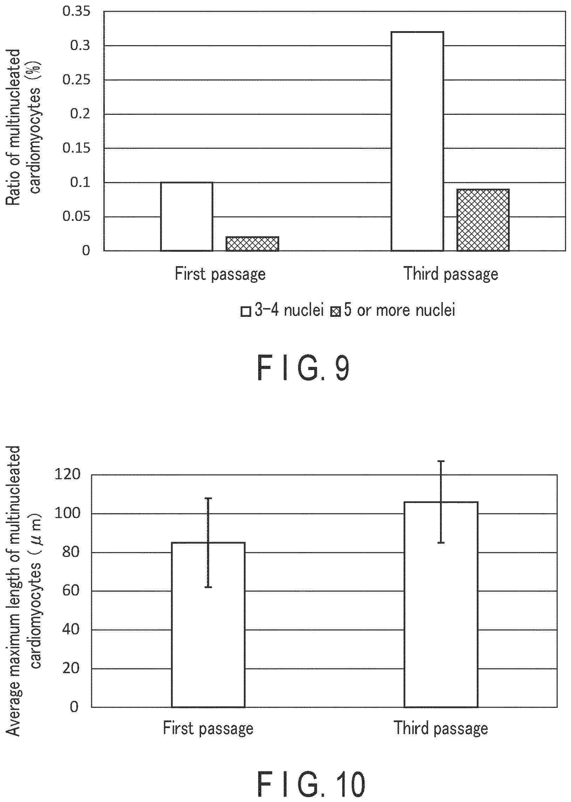

A ratio of multinucleated cardiomyocytes is shown in FIG. 9 and an average maximum length of the multinucleated cardiomyocytes is shown in FIG. 10.

When the seeding density of the cells was 2.0 × 10⁵ cells/cm² and the concentration of CHIR99021 was 3 µM, the cardiomyocyte population obtained after the first serial subculture contained multinucleated cardiomyocytes with three or four nuclei at a ratio of 0.1% and multinucleated cardiomyocytes with five or more nuclei at a ratio of 0.02% (see FIG. 9). That is, the obtained cardiomyocyte population contained multinucleated cardiomyocytes with three or more nuclei at a ratio of 0.12%. In this case, after the first serial subculture, the multinucleated cardiomyocytes (i.e., cardiomyocytes with two or more nuclei) had an average maximum length of 85 µm (see FIG. 10). In addition, striped sarcomeric structures were observed in the multinucleated cardiomyocytes after the first serial subculture.

When the seeding density of the cells was 2.0 × 10⁵ cells/cm² and the concentration of CHIR99021 was 3 µM, the cardiomyocyte population obtained after the third serial subculture contained multinucleated cardiomyocytes with three or four nuclei at a ratio of 0.32% and multinucleated cardiomyocytes with five or more nuclei at a ratio of 0.09% (see FIG. 9). That is, the obtained cardiomyocyte population contained multinucleated cardiomyocytes with three or more nuclei at a ratio of 0.41%. In this case, after the third serial subculture, the multinucleated cardiomyocytes (i.e., cardiomyocytes with two or more nuclei) had an average maximum length of 106 um (see FIG. 10). In addition, in the multinucleated cardiomyocytes after the third serial subculture, a clearer striped structure could be observed as compared with the multinucleated cardiomyocytes obtained after the first serial subculture.

From these results, it can be seen that when the number of passages of a serial subculture is increased, the ratio of multinucleated cardiomyocytes and the number of nuclei of multinucleated cardiomyocytes increase, and the size of multinucleated cardiomyocytes increases. It can also be seen that the degree of maturity of cardiomyocytes increases as the number of passages of a serial subculture increases.

### (c) Results when Using Commercially Available Cardiomyocytes

The present inventors used iCell2 of CDI (Cellular Dynamics International) Inc. instead of "cardiomyocytes induced from human iPS cells using a protein-free cardiomyocyte differentiation induction method" to perform culturing up to the fourth serial subculture according to the same method as in Example 2, and obtained results similar to those obtained when using the "cardiomyocytes induced from human iPS cells using a protein-free cardiomyocyte differentiation induction method".

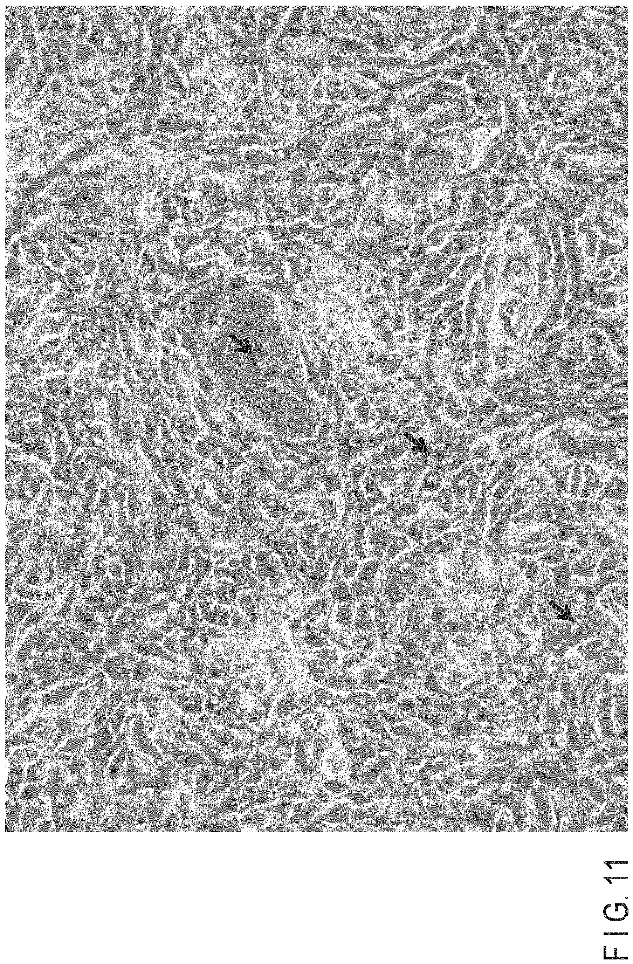

The cardiomyocyte population obtained using iCell2 of CDI Inc. is shown in FIG. 11. In FIG. 11, multinucleated cardiomyocytes are indicated by arrows. The multinucleated cardiomyocytes were large in size and prominent in the cardiomyocyte population. Thus, the methods described herein were shown to be applicable to all types of cardiomyocytes.

### [Example 3]

In Example 3, an effect of addition of an EGF receptor inhibitor (AG1478) was examined.

### [3-1] Method

Differentiation induction of human iPS cells (253G1 strain, Riken BioResource Center) was performed using a protein-free cardiomyocyte differentiation induction method, and cardiomyocytes (cTnT positive rate: 70 to 90%) from the 37th day from a start of differentiation induction were prepared as follows.
Cardiomyocyte lot No. 72: cTnT positive rate 75.8%
Cardiomyocyte lot No. 73: cTnT positive rate 88.2%
Cardiomyocyte lot No. 80: cTnT positive rate 71%
Cardiomyocyte lot No. 81: cTnT positive rate 87.3%
Cardiomyocyte lot No. 86: cTnT positive rate 84.7%

The cardiomyocytes were dispersed with trypsin (Life Technology) and then suspended in a 20% FBS cardiomyocyte differentiation culture medium (cardiomyocyte lot Nos. 72, 73, 80, and 81 contain 3 µM Y-27632, 3 µM CHIR99021, and 6 µM AG1478; cardiomyocyte lot No. 86 contains 3 µM Y-27632 and 3 µM CHIR99021 but no AG1478) in a state of single cardiomyocytes. The suspended cardiomyocytes were seeded in T25 flasks (Iwaki) coated with iMatrix laminin511 (Nippi, Incorporated). The cardiomyocytes were seeded at a cell density of 5.0 × 10⁶ cells/T25 flask (i.e., 2.0 × 10⁵ cells/cm²).

The cell-containing culture solution was placed in the culture vessel so that a distance from a liquid surface of the culture solution to a bottom surface of the culture vessel was 7 mm.

On the day after the seeding (day 1), the culture medium was replaced with a 2% FBS cardiomyocyte differentiation culture medium (cardiomyocyte lot Nos. 72, 73, 80, and 81 contain 3 µM CHIR99021 and 6 µM AG1478; cardiomyocyte lot No. 86 contains 3 µM CHIR99021 but no AG1478). Thereafter, the culture medium was replaced every 3 to 4 days, and culturing was performed for 2 weeks (a first serial subculture).

Cardiomyocyte lot No. 73 was cultured to a fourth serial subculture. Specifically, the cells obtained in the previous culturing were dispersed using trypsin-EDTA, and the obtained single cardiomyocytes were seeded in a flask at the same cell density as in the previous culturing and a serial subculture was performed under the same condition as described above.

### [3-2] Analysis

For the cells obtained after each serial subculture, "cardiomyocyte purity", "cardiomyocyte proliferation rate", "ratio of multinucleated cardiomyocytes", and "average maximum length of multinucleated cardiomyocytes" were determined according to the same procedure as in Example 1.

### [3-3] Result

### (a) Results of Cardiomyocyte Proliferation Rate and Cardiomyocyte Purity

After the first serial subculture, the cardiomyocytes proliferated at the following proliferation rates.
Cardiomyocyte lot No. 72: cardiomyocyte proliferation rate 2.5-fold
Cardiomyocyte lot No. 73: cardiomyocyte proliferation rate 2.1-fold
Cardiomyocyte lot No. 80: cardiomyocyte proliferation rate 3.2-fold
Cardiomyocyte lot No. 81: cardiomyocyte proliferation rate 2.9-fold
Cardiomyocyte lot No. 86: cardiomyocyte proliferation rate 3.0-fold

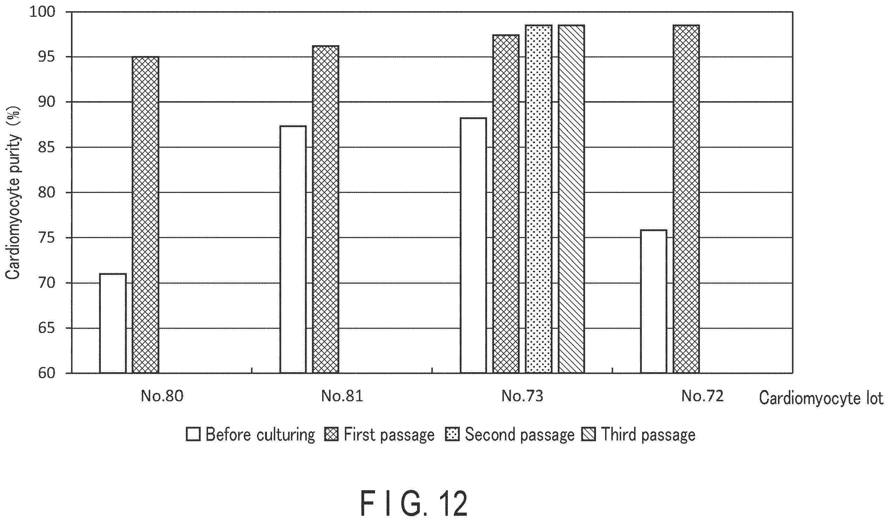

Results of cardiomyocyte purity are shown in FIG. 12.

Although the cardiomyocyte purity before the culturing was 70 to 90%, the cardiomyocyte purity (cTnT positive rate) of cardiomyocyte lot Nos. 72, 73, 80, and 81 were able to be increased to 95% or more after the first serial subculture (see FIG. 12). On the other hand, after the first serial subculture, the cardiomyocyte purity (cTnT positive rate) of cardiomyocyte lot No. 86 was 90%. In addition, in cardiomyocyte lot No. 73, a cardiomyocyte population having a cardiomyocyte purity of 98.5% at the maximum could be obtained after the third to fourth serial subcultures (see FIG. 12).

From the above results, it can be seen that when a cell population containing cardiomyocytes is seeded at an appropriate cell density and cultured by adding a WNT signal activator at an appropriate concentration (preferably 1 to 5 µM, more preferably 2 to 5 µM, still more preferably 2 to 4 µM, and most preferably 3 µM) and an EGF receptor inhibitor at an appropriate concentration (preferably 1 to 10 µM, more preferably 2 to 10 µM, still more preferably 4 to 8 µM, and most preferably 6 µM) to the culture solution, the cardiomyocyte purity can be significantly increased. It can also be seen that the cardiomyocyte purity can be brought close to 100% by increasing the number of passages of a serial subculture.

### (b) Result of Cardiomyocyte Population

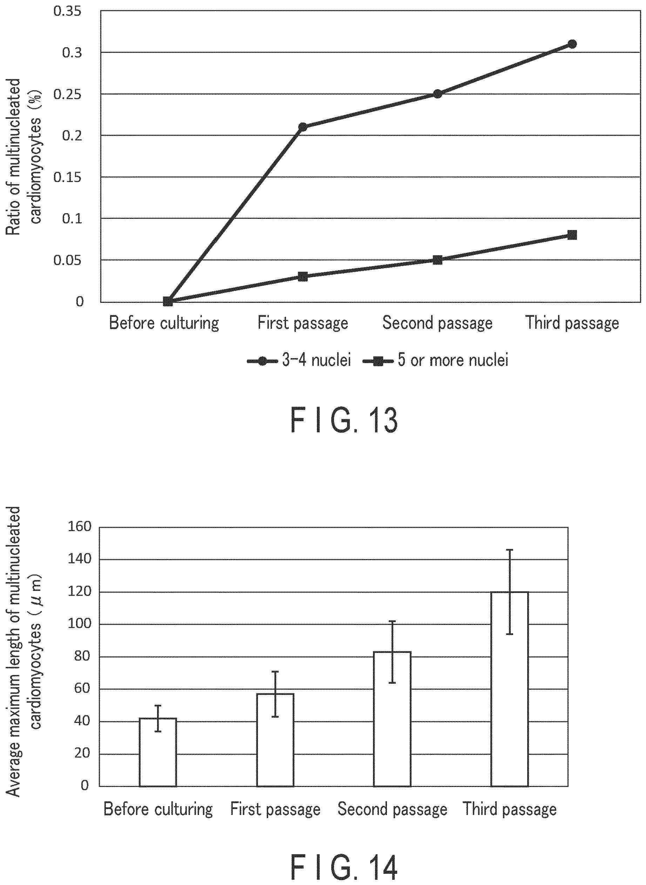

A ratio of multinucleated cardiomyocytes is shown in FIG. 13, and an average maximum length of the multinucleated cardiomyocytes is shown in FIG. 14.

In cardiomyocyte lot No. 73, the cardiomyocyte population obtained after the first serial subculture contained multinucleated cardiomyocytes with three or four nuclei at a ratio of 0.21% and multinucleated cardiomyocytes with five or more nuclei at a ratio of 0.03% (see FIG. 13). That is, the obtained cardiomyocyte population contained multinucleated cardiomyocytes with three or more nuclei at a ratio of 0.24%. In this case, after the first serial subculture, the multinucleated cardiomyocytes (i.e., cardiomyocytes with two or more nuclei) had an average maximum length of 57 µm (see FIG. 14). In addition, striped sarcomeric structures were observed in the multinucleated cardiomyocytes after the first serial subculture.

In cardiomyocyte lot No. 73, the cardiomyocyte population obtained after the third serial subculture contained multinucleated cardiomyocytes with three or four nuclei at a ratio of 0.31% and multinucleated cardiomyocytes with five or more nuclei at a ratio of 0.08% (see FIG. 13). That is, the obtained cardiomyocyte population contained multinucleated cardiomyocytes with three or more nuclei at a ratio of 0.39%. In this case, after the third serial subculture, the multinucleated cardiomyocytes (i.e., cardiomyocytes with two or more nuclei) had an average maximum length of 120 µm (see FIG. 14). In addition, in the multinucleated cardiomyocytes after the third serial subculture, more clear striped sarcomeric structures could be observed as compared with the multinucleated cardiomyocytes obtained after the first serial subculture.

From these results, it can be seen that when the number of passages of a serial subculture is increased, the ratio of multinucleated cardiomyocytes and the number of nuclei of multinucleated cardiomyocytes increase, and the size of multinucleated cardiomyocytes increases. It can also be seen that the degree of maturity of cardiomyocytes increases as the number of passages of a serial subculture increases.

### [Example 4]

Drug responsiveness of an obtained cardiomyocyte population was confirmed. Terfenadine was used as a drug. Terfenadine is an anti-allergy agent and is known to cause QT prolongation.

### [4-1] Method

According to the same method as in Example 3, cardiomyocytes were cultured to a third serial subculture to produce a cardiomyocyte population. The number of cardiomyocytes in the cardiomyocyte population increased about 15-fold from a start of a first serial subculture.

The obtained cardiomyocyte population was tested for drug responsiveness. A drug responsiveness test was performed by calcium imaging using a fluorescence microscope. Specifically, the drug responsiveness test was performed by acquiring pulsatile calcium fluorescence movies of cardiomyocytes on a fluorescence microscope Olympus IX83.

In the drug responsiveness test, a mixture having the following composition was adjusted to pH 7.4 and used as a culture solution for cardiomyocytes:
0.182 g/L of CaCl₂,
0.09767 g/L of MgSO₄,
0.4 g/L of KCl,
3.362 g/L of NaHCO₃,
5.4525 g/L of NaCl,
0.109 g/L of Na₂HPO₄,
5.958 g/L of HEPES, and
10% by mass of FBS (Fetal bovine serum, Invitrogen).

Terfenadine was added to the cell culture solution on a microscope, and after 10 minutes, a calcium signal was measured using calcium sensor protein GCaMP or calcium indicator Cal-520. ImageJ-based image analysis software was used for data analysis.

### [4-2] Result

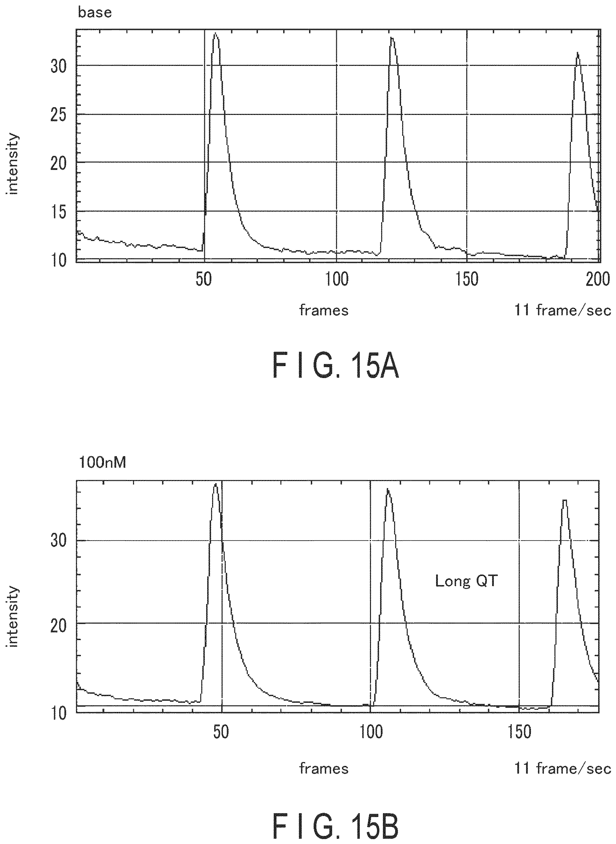

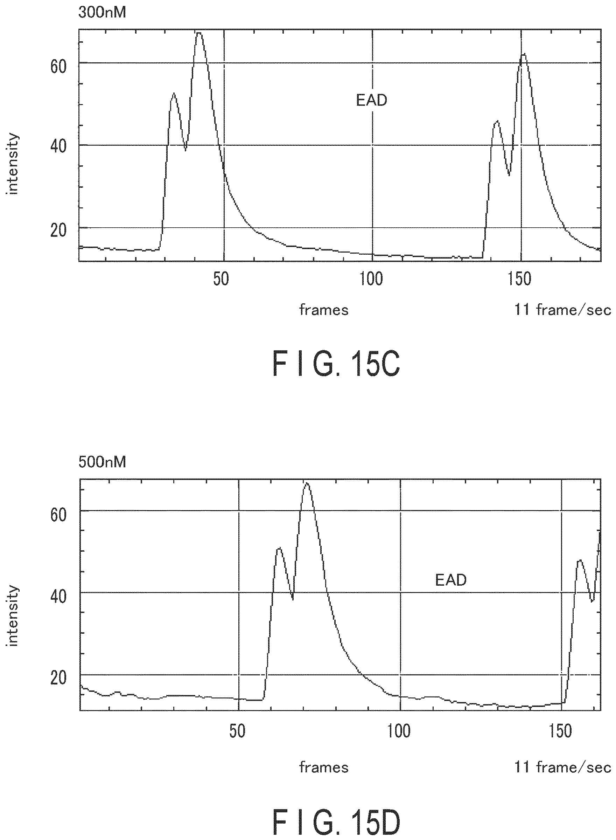

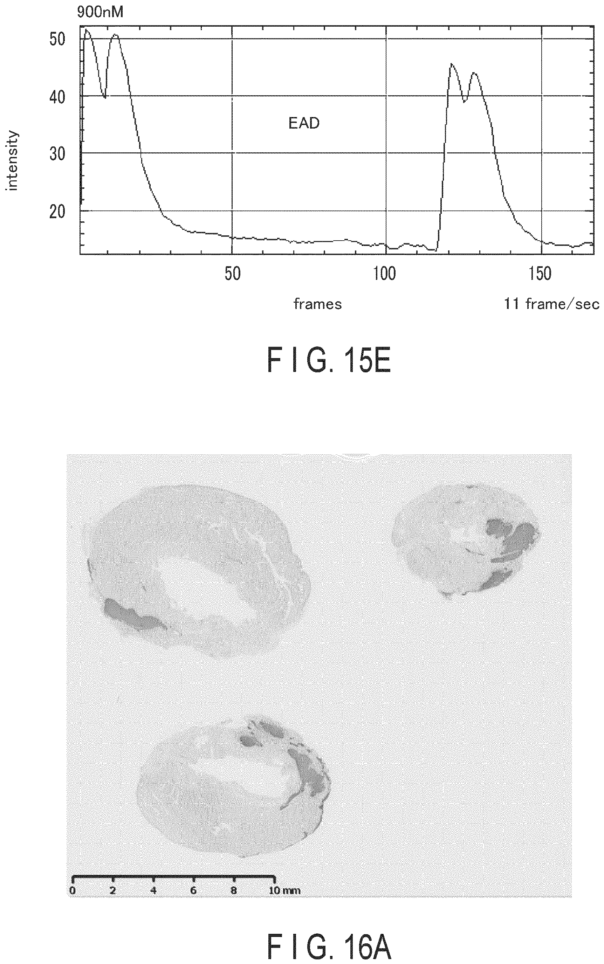

Measurement results are shown in FIGS. 15A to 15E. FIG. 15A is a waveform diagram when no terfenadine was added, FIG. 15B is a waveform diagram when terfenadine was added at a final concentration of 100 nM, FIG. 15C is a waveform diagram when terfenadine was added at a final concentration of 300 nM, FIG. 15D is a waveform diagram when terfenadine was added at a final concentration of 500 nM, and FIG. 15E is a waveform diagram when terfenadine was added at a final concentration of 900 nM.

A QT prolongation response was detected by the addition of terfenadine (concentration in culture solution: 100 nM) (see FIG. 15B). In addition, when the concentration of terfenadine was increased to 300 to 900 nM, an early afterdepolarization (EAD) response was detected (see FIGS. 15C to 15E). Therefore, it was confirmed that the obtained cardiomyocyte population maintained the function as cardiomyocytes.

### [Example 5]

An engraftment property of an obtained cardiomyocyte population was confirmed.

### [5-1] Method

According to the same method as in Example 3, cardiomyocytes were cultured to a second serial subculture to produce a cardiomyocyte population. The number of cardiomyocytes in the cardiomyocyte population increased about 4-fold from a start of a first serial subculture. The obtained cardiomyocyte population was transplanted into myocardial infarction rats.

### [5-2] Result

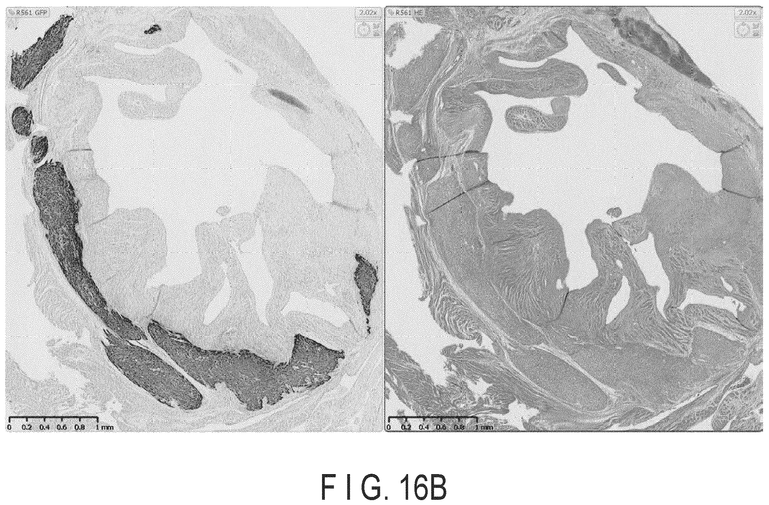

The hearts of the myocardial infarction rats after transplantation are shown in FIGS. 16A and 16B. A GFP gene was introduced into the transplanted cardiomyocyte population in advance. FIG. 16A shows results of staining with GFP antibodies. An upper part of FIG. 16B shows a result of staining of cardiomyocytes with myocardial markers, and a lower part shows a result of staining with GFP antibodies.

As shown in FIGS. 16A and 16B, when the obtained cardiomyocyte population was transplanted into the myocardial infarction rats, a large number of cardiomyocytes were engrafted, and engraftment of cells other than cardiomyocytes was not observed. Therefore, the obtained cardiomyocyte population can be expected to have high safety and a therapeutic effect in transplantation applications.

### [Example 6]

A cardiomyocyte population was produced according to the method of the present invention, and expression levels in a cardiomyocyte marker gene (cTnT) and an undifferentiated iPS cell marker gene (Lin28) were examined. The expression of an undifferentiated iPS cell marker gene (Lin28) indicates that iPS cells remain in the cardiomyocyte population. In addition, the cardiomyocyte population was subcutaneously transplanted into mice to evaluate tumorigenicity.

### [6-1] Method

### (Production of Cardiomyocyte Population)

Differentiation induction of human iPS cells (NH50191 strain, NIH, USA) was performed using a protein-free cardiomyocyte differentiation induction method, and cardiomyocytes (cTnT positive cell rate: 75.4%) from the 14th day from a start of differentiation induction were prepared.

The cardiomyocytes were dispersed with trypsin (Life Technology), and then suspended in a 20% FBS cardiomyocyte differentiation culture medium (containing 3 µM Y-27632, 2 µM CHIR99021, and 4 µM AG1478) in a state of single cardiomyocytes. For a composition of the "cardiomyocyte differentiation culture medium", the description in Example 1 can be referred to. The suspended cardiomyocytes were seeded in T25 flasks (Iwaki) coated with iMatrix laminin511 (Nippi, Incorporated). The cardiomyocytes were seeded at a cell density of 5.0 × 10⁶ cells/T25 flask (i.e., 2.0 × 10⁵ cells/cm²) .

On the day after the seeding (day 1), the culture medium was replaced with a 2% FBS cardiomyocyte differentiation culture medium (containing 2 µM CHIR99021 and 4 µM AG1478). Thereafter, the culture medium was replaced every 3 to 4 days, and culturing was performed for 2 weeks. Hereinafter, this culturing is referred to as an "extended culture". After the extended culture, the cells were dispersed using trypsin-EDTA to obtain cardiomyocytes in a single-cell state.

### (FACS Analysis)

For the obtained cardiomyocytes, the number of cells was counted, and FACS analysis of cTnT was performed. The number of cells was 18.5 × 10⁶ per T25 flask. A cTnT positive cell rate was 92.6%.

### (Marker Gene Expression Analysis)

Further, mRNA was recovered from the obtained cardiomyocytes (5 × 10⁷ cells) using an RNA recovery kit (miRNeasy Mini Kit, Qiagen). As comparative control 1, mRNA was recovered from undifferentiated iPS cells (1 × 10⁷ cells) using an RNA recovery kit (miRNeasy Mini Kit, Qiagen). As comparative control 2, mRNA was recovered using an RNA recovery kit (miRNeasy Mini Kit, Qiagen) from cardiomyocytes (without an extended culture) (5 × 10⁷ cells) on the 21st day after a start of the above-described differentiation induction. As comparative control 3, mRNA was recovered using an RNA recovery kit (miRNeasy Mini Kit, Qiagen) from cardiomyocytes (without an extended culture) (5 × 10⁷ cells) on the 28th day after the start of the above differentiation induction.
cDNA was synthesized from each mRNA with SuperScriptTM III reverse transcriptase. Gene expressions of cTnT, which is a cardiomyocyte marker gene, and Lin28, which is an undifferentiated iPS cell marker, were analyzed by a quantitative PCR method using PowerUp SYBR Green Master Mix (ThermoFisher) and QuantStudio 6 Flex Real-time PCR system (ThermoFisher). In this analysis, a gene expression of GAPDH was used as an internal standard. All PCR reactions were performed for 45 cycles. The above-described extended culture was performed three times independently. The three instances of extended cultures are referred to as "extended CM1", "extended CM2", and "extended CM3", respectively.

Sequences of primers used for the quantitative PCR are as follows:
Lin28_Fw: cacggtgcgggcatctg (sequence number 1)
Lin28_Rv: ccttccatgtgcagcttactc (sequence number 2)
cTnT_Fw: gaaggacctgaatgagttgcag (sequence number 3)
cTnT_Rv: acgtsctctcgatcctgtctttg (sequence number 4)

In sequence number 4, s represents g or c.

### (Evaluation of Tumorigenicity)

Tumorigenicity was evaluated for the cardiomyocytes obtained by performing an extended culture for 2 weeks, the above-described iPS cells (comparative control 1), and the cardiomyocytes on the 28th day after the start of differentiation induction (comparative control 3). Specifically, 1 × 10⁷ cells were subcutaneously injected into the back of NOG mice, and after 2 months, the presence or absence of a tumor was confirmed.

### [6-2] Result

### (FACS Analysis)

The two weeks of an extended culture allowed 18.5 × 10⁶ cardiomyocytes to be recovered per T25 flask. The number of cells increased 3.7-fold from 5 × 10⁶ cells at the time of seeding. After the extended culture for 2 weeks, the cTnT positive cell rate was 92.6%. The cTnT positive cell rate increased 17.2% from 75.4% at the time of seeding.

### (Marker Gene Expression Analysis)

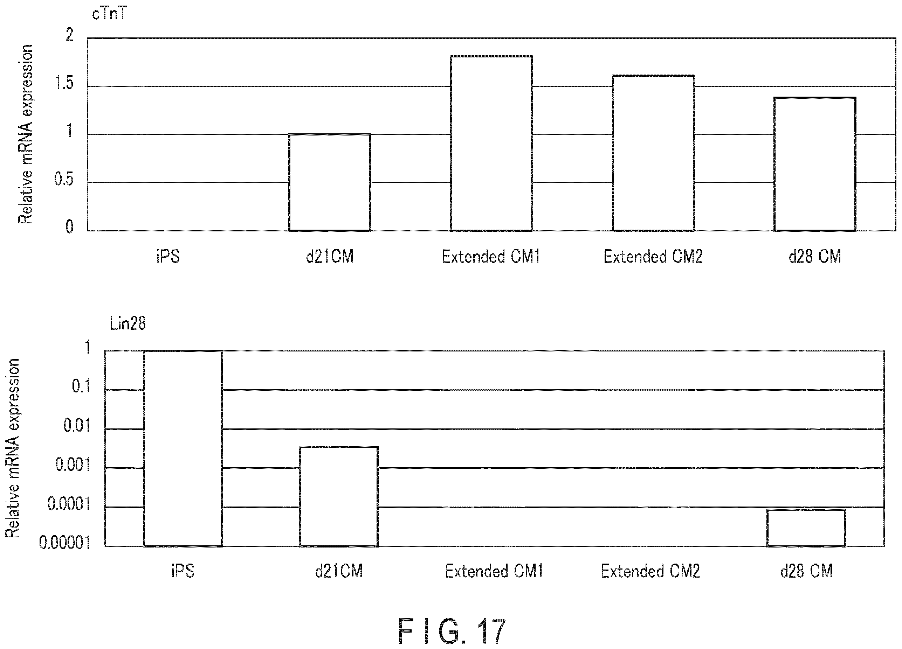

An analysis result of the cTnT gene expression is shown in an upper graph of FIG. 17. An analysis result of the Lin28 gene expression is shown in a lower graph of FIG. 17. Each graph in FIG. 17 shows results of the following samples in order from the left.
iPS: iPS cells (comparative control 1)
d21CM: cardiomyocytes on 21st day after start of differentiation induction (comparative control 2)
Extended CM1: cardiomyocytes obtained through an extended culture for 2 weeks
Extended CM2: cardiomyocytes obtained through an extended culture for 2 weeks
d28CM: cardiomyocytes on 28th day after start of differentiation induction (comparative control 3)

The upper graph shows relative cTnT expression levels when the cTnT expression level in d21CM (comparative control 3) is set to 1. The lower graph shows relative Lin28 expression levels when the Lin28 expression level in iPS (comparative control 1) is set to 1. The lower graph is a logarithmic graph.

As a result of the gene expression analysis, the expression of cTnT was not observed in the iPS cells, and the cardiomyocytes showed the same level of cTnT expression regardless of whether the extended culture was performed or not (the upper graph in FIG. 17). On the other hand, the expression of Lin28 was detected in the iPS cells. In the cardiomyocytes (d21CM) on the 21st day for which an extended culture was not performed and the cardiomyocytes (d28CM) on the 28th day for which an extended culture was not performed, Lin28 expression levels of about 0.1% to 0.01% of the iPS cells were shown. In the cardiomyocytes obtained through the extended culture (extended CM1 and extended CM2), the expression of Lin28 was not detected at all (the lower graph in FIG. 7).

Furthermore, the following experiment was performed to determine a detection limit of iPS cells remaining in the cardiomyocyte population. The RNA samples from the undifferentiated iPS cells were diluted to correspond to the certain numbers of cells (i.e., 1 × 10², 1 × 10³, 1 × 10⁴, 1 × 10⁷) and Lin28 expression level analysis was performed by quantitative PCR. This analysis was performed by simultaneous comparison with the RNA samples from the cardiomyocytes (5 × 10⁷ cell counts) obtained through the extended culture.

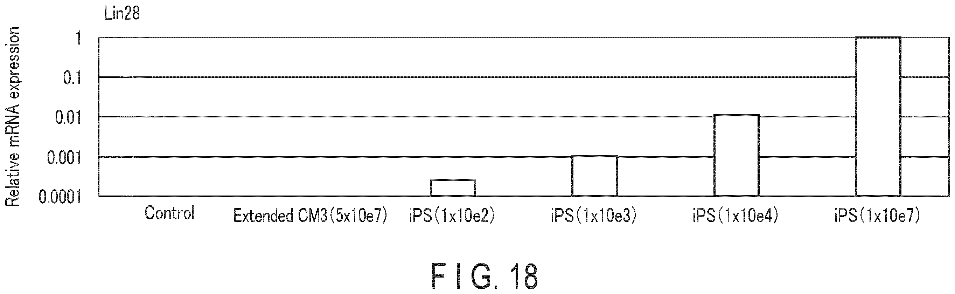

An analysis result of the Lin28 gene expression is shown in FIG. 18. The graph of FIG. 18 shows results of the following samples in order from the left.
Control: sample of PCR buffer alone (without cells) Extended CM3: RNA sample from cardiomyocytes obtained through an extended culture for 2 weeks
iPS (1 × 10e2): RNA sample from iPS cells being diluted to correspond to 1 × 10² iPS cell counts
iPS (1 × 10e3): RNA sample from iPS cells being diluted to correspond to 1 × 10³ iPS cell counts
iPS (1 × 10e4): RNA sample from iPS cells being diluted to correspond to 1 × 10⁴ iPS cell counts
iPS (1 × 10e7): RNA sample from iPS cells (comparative control 1)

The graph of FIG. 18 shows relative Lin28 expression levels when the Lin28 expression level in the iPS cells (comparative control 1) is set to 1. Note that this graph is a logarithmic graph.

Lin28 could be detected even when the RNA samples from the iPS cells were diluted to correspond to 1 × 10² iPS cell counts. On the other hand, Lin28 was not detected in the control and the cardiomyocytes after the extended culture (FIG. 18). From these results, it can be seen that the remaining number of iPS cells is less than 1 × 10² in the cardiomyocytes obtained by performing the extended culture for 2 weeks. This remaining number, when converted into a ratio of the number of cardiomyocytes (i.e., 5 × 10⁷ cell counts), is less than 0.0002%.

From the above, it can be seen that when the ratio of iPS cells remaining in the cardiomyocyte population is 0.0002% or more, the iPS cells can be detected by the quantitative PCR method.

### (Evaluation of Tumorigenicity)

An evaluation result of tumorigenicity will be described below. When iPS cells (comparative control 1) were subcutaneously transplanted into NOG mice, large tumors (2 to 5 mm) were generated in 4 out of 6 mice 2 months later. When the cardiomyocytes (comparative control 3) on the 28th day after the start of differentiation induction were subcutaneously transplanted into NOG mice, relatively small tumors (1 to 2 mm) were generated in 2 out of 6 mice 2 months later. When the cardiomyocytes obtained through the extended culture for 2 weeks were subcutaneously transplanted into NOG mice, no tumor was observed in all the 6 mice 2 months later.

These results indicate that the cardiomyocyte population produced by the method of the present invention is less prone to tumor formation.

### [6-3] Discussion

In regenerative medicine, tumorigenicity of transplanted cells is an important problem. It is known that transplantation of iPS cells in an undifferentiated state into an animal results in tumor formation, and it is desirable that the differentiated cells to be transplanted do not contain iPS cells in an undifferentiated state as much as possible. However, at present, in many cases, a certain number of undifferentiated cells remain in various differentiated cells induced to differentiate from iPS cells (e.g., cardiomyocytes induced to differentiate from iPS cells), which is a problem in regenerative medicine.

As a method of evaluating undifferentiated iPS cells remaining in differentiated cells, a method is known in which a gene expression level in Lin28, which is an undifferentiated iPS cell marker, is used as an indicator. In the experimental data described above, in the cardiomyocytes for which an extended culture was not performed, the expression of a certain amount of Lin28 was detected, as is conventionally known. However, the expression of Lin28 was not detected at all in the cardiomyocytes obtained by performing an extended culture according to the method of the present invention. In addition, in the cardiomyocytes obtained by performing an extended culture according to the method of the present invention, tumor formation was not observed even after cell transplantation in NOG mice.

From the above results, it is considered that the cardiomyocyte population prepared by the method of the present invention contains almost no undifferentiated iPS cells and is unlikely to become a tumor.

## Claims

1. A method of producing a cardiomyocyte population, the method comprising culturing a cell population containing cardiomyocytes seeded at a cell density of 0.1 × 10⁵ to 5.0 × 10⁵ cells/cm² in a culture solution to proliferate the cardiomyocytes.

2. The method according to claim 1, wherein the cell density is 2.0 × 10⁵ to 5.0 × 10⁵ cells/cm², and preferably 2.0 × 10⁵ to 4.0 × 10⁵ cells/cm².

3. The method according to claim 1, wherein the culture solution contains a WNT signal activator.

4. The method according to claim 3, wherein the cell density is 0.3 × 10⁵ to 4.0 × 10⁵ cells/cm², and preferably 1.0 × 10⁵ to 3.0 × 10⁵ cells/cm².

5. The method according to any one of claims 1 to 4, wherein the culturing is performed under a condition in which a distance from a liquid surface of the culture solution to a culture vessel bottom surface in contact with the cardiomyocytes is 3 to 30 mm.

6. The method according to any one of claims 1 to 5, wherein the culture solution contains an EGF receptor inhibitor.

7. The method according to any one of claims 1 to 6, wherein the culture solution contains serum.

8. The method according to any one of claims 1 to 7, wherein the culturing is performed through a serial subculture.

9. The method according to any one of claims 1 to 8, wherein the cell population containing cardiomyocytes is a cell population containing human cardiomyocytes.

10. The method according to any one of claims 1 to 9, wherein the cell population containing cardiomyocytes is a cell population obtained by inducing differentiation of pluripotent stem cells into cardiomyocytes.

11. A method of proliferating cardiomyocytes, the method comprising culturing a cell population containing cardiomyocytes seeded at a cell density of 0.1 × 10⁵ to 5.0 × 10⁵ cells/cm² in a culture solution to proliferate the cardiomyocytes.

12. A cardiomyocyte population containing human multinucleated cardiomyocytes including three or more nuclei and derived from pluripotent stem cells.

13. A transplantation material comprising a cardiomyocyte population containing human multinucleated cardiomyocytes including three or more nuclei and derived from pluripotent stem cells.

14. A human multinucleated cardiomyocyte with five or more nuclei.

15. A cardiomyocyte population containing human multinucleated cardiomyocytes with five or more nuclei.

16. A transplantation material comprising a cardiomyocyte population containing human multinucleated cardiomyocytes with five or more nuclei.

17. The cardiomyocyte population according to claim 12 or 15, wherein the cardiomyocyte population has a cardiomyocyte purity of 80% or more.

18. The cardiomyocyte population according to claim 12, 15, or 17, wherein the human multinucleated cardiomyocytes have an average maximum length of 50 to 1000 pm, when measured in a state of being adhered to a culture vessel bottom surface.

19. The cardiomyocyte population according to claim 12, 15, 17, or 18, wherein the human multinucleated cardiomyocytes include developed muscle fibers.

20. The transplantation material according to claim 13 or 16, wherein the cardiomyocyte population has a cardiomyocyte purity of 80% or more.

21. The transplantation material according to claim 13, 16, or 20, wherein the human multinucleated cardiomyocytes have an average maximum length of 50 to 1000 pm, when measured in a state of being adhered to a culture vessel bottom surface.

22. The transplantation material according to claim 13, 16, 20, or 21, wherein the human multinucleated cardiomyocytes include developed muscle fibers.

23. A cardiomyocyte population produced by the method according to any one of claims 1 to 10.

24. A transplantation material comprising a cardiomyocyte population produced by the method according to any one of claims 1 to 10.

25. A kit for proliferating cardiomyocytes, the kit comprising a cell population containing cardiomyocytes, a culture solution for cardiomyocytes, a WNT signal activator, and an EGF receptor inhibitor.
